# EUROPEAN PATENT APPLICATION

(11) **EP 4 825 387 A1**
(43) Date of publication of application: **21.10.2026**
(21) Application number: 24904468.6
(22) Date of filing: 16.12.2024
(51) Int. Cl.: A61K 38/00, A61K 38/20, A61K 38/17, A61K 40/42, A61K 40/31, A61K 40/11, A61P 35/00

(54) **FUSION PROTEIN COMPRISING CD80 PROTEIN AND IL-2 PROTEIN, AND COMPOSITION FOR PREVENTING OR TREATING CANCER COMPRISING T CELLS EXPRESSING CHIMERIC ANTIGEN RECEPTORS**

(30) Priority: 14.12.2023 KR 20230181709; 05.11.2024 KR 20240155086
(71) Applicant: GI Innovation, Inc., Seoul 05855 (KR)
(72) Inventor: JANG, Myung Ho, Seoul 05855 (KR); KOH, Young Jun, Seoul 05855 (KR); YUN, Nari, Seoul 05855 (KR); PARK, Min, Seoul 05855 (KR); LEE, Hae Jong, Seoul 05855 (KR)
(74) Representative: HGF
(86) International application number: PCT/KR2024/097036
(87) International publication number: WO 2025/127905

(57) **Abstract**

The present invention relates to a pharmaceutical composition for the prevention or treatment of cancer comprising a fusion protein containing the IL-2 protein and the CD80 protein, and CAR-expressing immune cells. When the fusion protein of the present invention is co-administered with chimeric antigen receptor (CAR)-expressing immune cells, specifically CAR-T cells, not only is the immune activity of the CAR-T cells within the tumor enhanced and sustained, but when the fusion protein of the present invention is administered alone to subjects in whom cancer has recurred after CAR-T cell administration, it can eliminate recurrent cancer cells by inducing the reactivation of previously administered CAR-T cells, thereby eliminating the recurrent cancer cells. Furthermore, even when cancer cells are re-implanted into an animal model in which cancer cells were completely eliminated by combined treatment of CAR-T cells and the fusion protein of the present invention, the immune activity of the CAR-T cells is maintained for a long period by the fusion protein, continuously suppressing the proliferation of the re-implanted cancer cells. Thus, the fusion protein of the present invention not only enhances and prolongs the anti-tumor immune activity of CAR-T cells but also maximizes the efficiency of CAR-T cells as a therapeutic cell resource by repeatedly reactivating previously administered CAR-T cells, thereby significantly improving survival rates of the patient.

## Description

### Technical Field

The present invention relates to a pharmaceutical composition for the treatment of cancer comprising a fusion protein comprising CD80 and IL-2 proteins and T cells expressing a chimeric antigen receptor.

### Background Art

Interleukin-2 (hereinafter IL-2) is a cytokine released by T cells that promotes T cell proliferation and differentiation. The IL-2 and IL-2R system is known to promote the proliferation and anticancer activity of CAR (chimeric antigen receptor)-T cells (Zhang et al., Sci Transl Med., 13(625), 2021). However, it has been reported that the use of IL-2 carries the risk of regulatory T cell proliferation, which may interfere with anticancer responses (Bell et al., Function. Front Immunol. 12:684642, 2021).

Meanwhile, the human CD19 molecule is a cell surface receptor expressed on the surface of human B cells, such as pre-B cells, immature B cells in early stages of development, mature B cells, and malignant B cells. Consequently, most B-cell lineage malignancies, including non-Hodgkin's lymphoma, chronic lymphocytic leukemia (CLL), and acute lymphoblastic leukemia (ALL), express CD19. Despite recent active research into various anticancer agents, tumors expressing CD19 have a poor prognosis and do not respond well to conventional treatments, so there remains a high demand for the development of improved treatment methods or therapeutic compositions.

CAR-T therapies targeting the CD19 antigen demonstrate excellent antitumor efficacy against various B-cell lymphomas, including diffuse large B-cell lymphoma (DLBCL), and significantly improve survival rates; however, it is known that disease progresses or recurs in approximately 60% of patients who receive CAR-T cells. In this regard, strategies are needed to improve the long-term therapeutic efficacy of CAR-T cells.

Throughout the present specification, a number of publications and patent documents are referred to and cited. The disclosure of the cited publications and patent documents is incorporated herein by reference in its entirety to more clearly describe the state of the art to which the present invention pertains and the content of the present invention.

### DISCLOSURE

### Technical Problem

The present inventors have made intensive studies to develop an effective combination therapy capable of significantly improving the anticancer activity and persistence of immune cell therapy. As a result, the present inventors completed the present invention by demonstrating that a fusion protein comprising CD80 and IL-2 not only significantly enhances the immune activity of immune cells expressing a chimeric antigen receptor (CAR), specifically CAR-T cells, but also enables the long-term maintenance and repeated reactivation of such activity.

Accordingly, it is an object of the present invention to provide a pharmaceutical composition for the preventing or treating cancer comprising a fusion protein comprising a CD80 protein and an IL-2 protein, and CAR-expressing immune cells.

It is another object of the present invention to provide an anticancer composition for co-administration with CAR-expressing immune cells, comprising a fusion protein comprising a CD80 protein and an IL-2 protein.

It is another object of the present invention to provide a composition for enhancing the activity of CAR-expressing immune cells, comprising a fusion protein comprising a CD80 protein and an IL-2 protein.

Other objects and advantages of the present invention will become more apparent from the following detailed description, the appended claims, and the accompanying drawings.

### Technical Solution

In one aspect of this invention, there is provided a composition for enhancing the activity of an immune cell that expresses a chimeric antigen receptor, comprising as an active ingredient, a fusion protein comprising a CD80 protein or a fragment thereof and an IL-2 protein or a variant thereof.

In another aspect of this invention, there is provided a method for enhancing the activity of an immune cell that expresses a chimeric antigen receptor, comprising administering to a subject in need thereof a fusion protein comprising a CD80 protein or a fragment thereof and an IL-2 protein or a variant thereof.

As used herein, the term "fusion protein" refers to a recombinant protein molecule in which an amino acid sequence derived from a specific protein or domain is fused to another amino acid sequence derived from a protein or domain of a different origin. Each amino acid sequence of different origin may be directly linked within the fusion protein or may be linked via a linker sequence, a tag sequence, a self-cleaving sequence, or a combination thereof.

As used herein, the term "IL-2 (interleukin-2) refers to any wild-type IL-2 obtained from any vertebrate source, including mammals, for example, primates and rodents. IL-2 may be obtained from animal cells, and also includes one obtained from recombinant cells capable of producing IL-2. In addition, IL-2 may be wild-type IL-2 or a variant thereof. IL-2 may be in a mature form. Specifically, the mature IL-2 may not include a signal sequence and may have the amino acid sequence of SEQ ID NO: 10. In this case, "IL-2" may be used to refer to a fragment in which a portion of the N-terminus or C-terminus of wild-type IL-2 is truncated.

As used herein, the term "IL-2 variant" refers to a form in which a portion of the amino acid sequence of full-length IL-2 or a fragment thereof has been substituted. However, said IL-2 variant may possess activity equivalent to or similar to that of wild-type IL-2. Here, "IL-2 activity" may mean, for example, specific binding to the IL-2 receptor, which may be measured by methods known in the art.

Specifically, an IL-2 variant may be obtained by substitution of a portion of amino acids in the wild-type IL-2. An embodiment of the IL-2 variant obtained by amino acid substitution may be obtained by substitution of at least one of the 38^{th}, 42^{nd}, 45^{th}, 61^{st}, and 72^{nd} amino acids in the amino acid sequence of SEQ ID NO: 10.

More specifically, the variant of the IL-2 protein may be a variant comprising a substitution of one or more of the 38^{th}, 42^{nd}, 45^{th}, 61^{st} and 72^{nd} amino acid in the amino acid sequence of SEQ ID NO: 10.

In an embodiment, an IL-2 variant may be in a form in which two amino acids are substituted. Specifically, the IL-2 variant may be obtained by substitution of the 38^{th} and 42^{nd} amino acids in the amino acid sequence of SEQ ID NO: 10. In addition, in an embodiment, the IL-2 variant may be obtained by substitution of the 38^{th} and 61^{st} amino acids in the amino acid sequence of SEQ ID NO: 10. In addition, in an embodiment, the IL-2 variant may be obtained by substitution of the 38^{th} and 72^{nd} amino acids in the amino acid sequence of SEQ ID NO: 10. In addition, in an embodiment, the IL-2 variant may be obtained by substitution of the 42^{nd} and 45^{th} amino acids in the amino acid sequence of SEQ ID NO: 10. In addition, in an embodiment, the IL-2 variant may be obtained by substitution of the 42^{nd} and 61^{st} amino acids in the amino acid sequence of SEQ ID NO: 10. In addition, in an embodiment, the IL-2 variant may be obtained by substitution of the 42^{nd} and 72^{nd} amino acids in the amino acid sequence of SEQ ID NO: 10. In addition, in an embodiment, the IL-2 variant may be obtained by substitution of the 45^{th} and 61^{st} amino acids in the amino acid sequence of SEQ ID NO: 10. In addition, in an embodiment, the IL-2 variant may be obtained by substitution of the 45^{th} and 72^{nd} amino acids in the amino acid sequence of SEQ ID NO: 10. In addition, in an embodiment, the IL-2 variant may be obtained by substitution of the 61^{st} and 72^{nd} amino acids in the amino acid sequence of SEQ ID NO: 10.

Furthermore, an IL-2 variant may be in a form in which three amino acids are substituted. Specifically, the IL-2 variant may be obtained by substitution of the 38^{th}, 42^{nd}, and 45^{th} amino acids in the amino acid sequence of SEQ ID NO: 10. In addition, in an embodiment, the IL-2 variant may be obtained by substitution of the 38^{th}, 42^{nd} and 61^{st} amino acids in the amino acid sequence of SEQ ID NO: 10. In addition, in an embodiment, the IL-2 variant may be obtained by substitution of the 38^{th}, 42^{nd} and 72^{nd} amino acids in the amino acid sequence of SEQ ID NO: 10. In addition, in an embodiment, the IL-2 variant may be obtained by substitution of the 38^{th}, 45^{th} and 61^{st} amino acids in the amino acid sequence of SEQ ID NO: 10. In addition, in an embodiment, the IL-2 variant may be obtained by substitution of the 38^{th}, 45^{th} and 72^{nd} amino acids in the amino acid sequence of SEQ ID NO: 10. In addition, in an embodiment, the IL-2 variant may be obtained by substitution of the 38^{th}, 61^{st}, and 72^{nd} amino acids in the amino acid sequence of SEQ ID NO: 10. In addition, in an embodiment, the IL-2 variant may be obtained by substitution of the 42^{nd}, 45^{th} and 61^{st} amino acids in the amino acid sequence of SEQ ID NO: 10. In addition, in an embodiment, the IL-2 variant may be obtained by substitution of the 42^{nd}, 45^{th} and 72^{nd} amino acids in the amino acid sequence of SEQ ID NO: 10. In addition, in an embodiment, the IL-2 variant may be obtained by substitution of the 45^{th}, 61^{st} and 72^{nd} amino acids in the amino acid sequence of SEQ ID NO: 10.

In this case, the "different amino acids" introduced at the listed residue positions may be any one selected from the group consisting of alanine, arginine, asparagine, aspartic acid, cysteine, glutamic acid, glutamine, histidine, isoleucine, leucine, lysine, methionine, phenylalanine, proline, serine, threonine, tryptophan, tyrosine, and valine. However, regarding amino acid substitution for the IL-2 variant, in the amino acid sequence of SEQ ID NO: 10, the 38^{th} amino acid cannot be substituted with arginine, the 42^{nd} amino acid cannot be substituted with phenylalanine, the 45^{th} amino acid cannot be substituted with tyrosine, the 61^{st} amino acid cannot be substituted with glutamic acid, and the 72^{nd} amino acid cannot be substituted with leucine.

Regarding amino acid substitution for an IL-2 variant, the 38^{th} amino acid of SEQ ID NO: 10, arginine, may be substituted with an amino acid other than arginine. Preferably, regarding amino acid substitution for an IL-2 variant, the 38^{th} amino acid of SEQ ID NO: 10, arginine, may be substituted with alanine (R38A).

Regarding amino acid substitution for an IL-2 variant, the 42^{nd} amino acid of SEQ ID NO: 10, phenylalanine, may be substituted with an amino acid other than phenylalanine. Preferably, regarding amino acid substitution for an IL-2 variant, the 42^{nd} amino acid of SEQ ID NO: 10, phenylalanine, may be substituted with alanine (F42A).

Regarding amino acid substitution for an IL-2 variant, the 45^{th} amino acid of SEQ ID NO: 10, tyrosine, may be substituted with an amino acid other than tyrosine. Preferably, regarding amino acid substitution for an IL-2 variant, the 45^{th} amino acid of SEQ ID NO: 10, tyrosine, may be substituted with alanine (Y45A).

Regarding amino acid substitution for an IL-2 variant, the 61^{st} amino acid of SEQ ID NO: 10, glutamic acid, may be substituted with an amino acid other than glutamic acid. Preferably, regarding amino acid substitution for an IL-2 variant, the 61^{st} amino acid of SEQ ID NO: 10, glutamic acid, may be substituted with arginine (E61R).

Regarding amino acid substitution for an IL-2 variant, the 72^{nd} amino acid of SEQ ID NO: 10, leucine, may be substituted with an amino acid other than leucine. Preferably, regarding amino acid substitution for an IL-2 variant, the 72^{nd} amino acid of SEQ ID NO: 10, leucine, may be substituted with glycine (L72G).

Specifically, an IL-2 variant may comprise at least one substitution selected from the group consisting of R38A, F42A, Y45A, E61R, and L72G, in the amino acid sequence of SEQ ID NO: 10.

Specifically, an IL-2 variant may comprise two or three substitutions selected from the group consisting of R38A, F42A, Y45A, E61R, and L72G.

In addition, an IL-2 variant may be in a form in which two amino acids are substituted. Specifically, an IL-2 variant may comprise substitutions of R38A and F42A. In addition, in an embodiment, an IL-2 variant may comprise substitutions of R38A and Y45A. In addition, in an embodiment, an IL-2 variant may comprise substitutions of R38A and E61R. In addition, in an embodiment, an IL-2 variant may comprise substitutions of R38A and L72G. In addition, in an embodiment, an IL-2 variant may comprise substitutions of F42A and Y45A. In addition, in an embodiment, an IL-2 variant may comprise substitutions of F42A and E61R. In addition, in an embodiment, an IL-2 variant may comprise substitutions of F42A and L72G. In addition, in an embodiment, an IL-2 variant may comprise substitutions of E61R and L72G.

Furthermore, an IL-2 variant may be in a form in which three amino acids are substituted. Specifically, an IL-2 variant may may comprise substitutions of R38A, F42A and Y45A. In addition, in an embodiment, an IL-2 variant may comprise substitutions of R38A, F42A and E61R. In addition, in an embodiment, an IL-2 variant may comprise substitutions of R38A, F42A and L72G. In addition, in an embodiment, an IL-2 variant may comprise substitutions of R38A, Y45A and E61R. In addition, in an embodiment, an IL-2 variant may comprise substitutions of R38A, Y45A and L72G. In addition, in an embodiment, an IL-2 variant may comprise substitutions of F42A, Y45A and E61R. In addition, in an embodiment, an IL-2 variant may comprise substitutions of F42A, Y45A and L72G. In addition, in an embodiment, an IL-2 variant may comprise substitutions of F42A, E61R and L72G. In addition, in an embodiment, an IL-2 variant may comprise substitutions of Y45A, E61R and L72G.

In addition, an IL-2 variant may be in a form in which four amino acids are substituted. Specifically, an IL-2 variant may comprise substitutions of R38A, F42A, Y45A and E61R. In addition, in an embodiment, an IL-2 variant may comprise substitutions of R38A, F42A, Y45A and L72G. In addition, in an embodiment, an IL-2 variant may comprise substitutions of R38A, F42A, E61R and L72G. In addition, in an embodiment, an IL-2 variant may comprise substitutions of R38A, Y45A, E61R and L72G. In addition, in an embodiment, an IL-2 variant may comprise substitutions of F42A, Y45A, E61R and L72G.

Furthermore, an IL-2 variant may comprise substitutions of R38A, F42A, Y45A, E61R, and L72G.

Specifically, the IL-2 variant may comprise one or more substitutions selected from the group consisting of R38A, F42A, and E61R in the amino acid sequence of SEQ ID NO: 10. More specifically, the IL-2 variant may comprise substitutions of R38A, F42A, and E61R in the amino acid sequence of SEQ ID NO: 10.

The aforementioned IL-2 variants of the present invention exhibit reduced in vivo toxicity compared to wild-type IL-2 due to binding to the alpha subunit of the IL-2 receptor (IL-2Rα).

As used herein, the term "CD80", also called "B7-1", is a membrane protein present in dendritic cells, activated B cells, and monocytes. CD80 provides co-stimulatory signals essential for activation and survival of T cells. CD80 is composed of 288 amino acids, and is known as a ligand for the two different proteins, CD28 and CTLA-4.

As used herein, the term "CD80 fragment" refers a fragment of the full-length CD80 protein in which some amino acid residues have been deleted, yet retains the inherent biological activity and function of the full-length protein. Specifically, it refers to the functional fragment of CD80 capable of transmitting a costimulatory signal for T cell activation. For example, the functional fragment of CD80 may be the extracellular domain of CD80 or a fragment of the full-length CD80 protein containing said domain.

An embodiment of the CD80 fragment may be a fragment in which the 1^{st} to 34^{th} amino acids from N-terminus which are a signal sequence of CD80, are removed. Specifically, the CD80 fragment may be a fragment consisting of the 35^{th} to 288^{th} amino acids in SEQ ID NO: 11. In addition, the CD80 fragment may be a fragment consisting of the 35^{th} to 242^{nd} amino acids in SEQ ID NO: 11. In addition, the CD80 fragment may be a fragment consisting of the 35^{th} to 232^{nd} amino acids in SEQ ID NO: 11. In addition, the CD80 fragment may be a fragment consisting of the 35^{th} to 139^{th} amino acids in SEQ ID NO: 11. In addition, the CD80 fragment may be a fragment consisting of the 142^{nd} to 242^{nd} amino acids in SEQ ID NO: 11.

More specifically, the fragment of the CD80 protein may be the extracellular domain of the CD80 protein. Even more specifically, the CD80 fragment may have the amino acid sequence of SEQ ID NO: 2.

According to a specific embodiment, the chimeric antigen receptor expressed by the immune cells of the present invention comprises all or portion of an intracellular signaling domain selected from the group consisting of CD3z, CD28 and 4-1BB.

The term "portion" as used herein in reference to a specific protein or domain refers to a fragment of the full-length protein from which some amino acid residues have been deleted, yet which retains the inherent biological activity and function of the full-length protein. Therefore, the term "portion" has the same meaning as "functional portion."

According to a specific embodiment, the chimeric antigen receptor comprises a transmembrane domain comprising all or portion of CD28 or CD8.

According to a specific embodiment of the present invention, the chimeric antigen receptor comprises a hinge domain comprising all or a portion of the CD8 hinge or the CD28 hinge.

According to a specific embodiment, the chimeric antigen receptor comprises an extracellular antigen-binding domain that binds to CD19 or BCMA (B-cell maturation antigen).

More specifically, the chimeric antigen receptor of the present invention may comprise the CD8 hinge domain, the CD28 transmembrane domain, and the CD3ζ intracellular signaling domain sequentially arranged from the N-terminus to the C-terminus. Most specifically, the chimeric antigen receptor of the present invention may comprise the hinge domain, transmembrane domain, and intracellular signaling domain of ProMab^{®}anti-CD19 CAR (Cat.# PM-CAR1001).

More specifically, the chimeric antigen receptor of the present invention may comprise a CD8 hinge domain, a CD8 transmembrane domain, a 4-1BB intracellular signaling (costimulatory) domain, and a CD3ζ intracellular signaling domain sequentially arranged from the N-terminus to the C-terminus. Most specifically, the chimeric antigen receptor of the present invention may comprise the hinge domain, transmembrane domain, and intracellular signaling domain of Kymriah^{®} anti-CD19 CAR or Abecma^{®} anti-BCMA CAR.

More specifically, the chimeric antigen receptor of the present invention may comprise a CD8 hinge domain, a CD28 transmembrane domain, a CD28 intracellular signaling (costimulatory) domain, and a CD3ζ intracellular signaling domain sequentially arranged from the N-terminus toward the C-terminus. Most specifically, the chimeric antigen receptor of the present invention may comprise the hinge domain, transmembrane domain, and intracellular signaling domain of ProMab^{®} anti-BCMA CAR (Cat.# PM-CAR1031).

More specifically, the chimeric antigen receptor of the present invention may comprise the CD28 hinge domain, CD28 transmembrane domain, CD28 intracellular signaling (costimulatory) domain, and CD3ζ intracellular signaling domain sequentially arranged from the N-terminus to the C-terminus. Most specifically, the chimeric antigen receptor of the present invention may comprise the hinge domain, transmembrane domain, and intracellular signaling domain of the Yescarta^{®} anti-CD19 CAR.

More specifically, the chimeric antigen receptor of the present invention may comprise a CD8 hinge domain, a CD28 transmembrane domain, a 41BB intracellular signaling (co-stimulatory) domain, and a CD3ζ intracellular signaling domain arranged sequentially from the N-terminus to the C-terminus. Most specifically, the chimeric antigen receptor of the present invention may comprise the hinge domain, transmembrane domain, and intracellular signaling domain of ProMab^{®} anti-BCMA CAR (Cat.# PM-CAR1037).

As used herein, the term "extracellular antigen-binding domain" refers to a domain that is positioned in the extracellular portion when a chimeric antigen receptor is expressed on a cell (e.g., an immune cell), and specifically recognizes a target antigen to activate the cytotoxic activity of the immune cell in a target cell (e.g., cancer cells)-specific manner. For example, it may be an antigen-binding fragment of an antibody, such as an Fc receptor or a single-chain variable fragment (scFv). Accordingly, the term "extracellular antigen binding domain" as used herein is used interchangeably with "extracellular domain", "antigen-recognizing fragment" or "antigen-binding fragment".

As used herein, "specifically binds" or "specifically recognizes" means that an antibody or its antigen-binding fragment forms a stable complex with an antigen under physiological conditions with an affinity of at least a certain threshold. Specific binding can be defined, for example, by an equilibrium dissociation constant of at least approximately 1 ×10⁻⁶ M or less. Methods for determining whether two molecules bind specifically are well known in the art and include, for example, equilibrium dialysis and surface plasmon resonance.

As used herein, the term "affinity" refers to the strength of the sum of non-covalent interactions between a single binding site of a molecule (e.g., an antibody) and its binding partner (e.g., an antigen). Unless otherwise specified, "binding affinity" refers to the intrinsic binding affinity reflecting a 1:1 interaction between members of a binding pair (e.g., an antibody and an antigen). Affinity can generally be expressed as a dissociation constant (Kd) and can be measured by conventional methods known in the art.

According to a specific embodiment of the present invention, the extracellular antigen-binding domain used in the chimeric antigen receptor of the present invention is an antigen-binding fragment of an anti-CD19 antibody or an antigen-binding fragment of an anti-BCMA antibody.

As used herein, the term "antibody" refers to a peptide molecule that that specifically recognizes and binds to a specific epitope of CD19 or BCMA, and includes antigen-binding fragments of the antibody (immunologically active fragments) as well as the whole antibody molecule.

A complete antibody has a structure with two full-length light chains and two full-length heavy chains, which are linked to each other by a disulfide bond. The heavy chain constant region has the gamma (γ), mu (µ), alpha (α), delta (δ), and epsilon (ε) types and is subclassified into gamma1 (γ1), gamma2 (γ2), gamma3 (γ3), gamma4 (γ4), alpha1 (α1), and alpha2 (α2). The light chain constant region has kappa (κ) and lambda (λ) types.

As used herein, the term "antigen-binding fragment of the antibody" refers to a portion of a polypeptide that has significant antigen-antibody binding functionality within the overall structure of an immunoglobulin. Examples of the antigen-binding fragment include, but are not limited to, Fab fragment, F(ab') fragment, F(ab')2 fragment and Fv fragment.

Among antibody fragments, Fab is a structure with one antigen-binding site containing variable regions of the light and heavy chains, a constant region of the light chain and the first constant region of the heavy chain (CH1). Fab' differs from Fab in that it has a hinge domain containing one or more cysteine residues at the C-terminus of the heavy chain CH1 domain. F(ab')2 antibodies are generated by disulfide bond formed by cysteine residues in the hinge of Fab'. Fv is minimal antibody fragments that have only a heavy chain variable region and a light chain variable region. Two-chain Fv has a heavy chain variable region and a light chain variable region linked by non-covalent bonds, while a single-chain Fv (single-chain Fv, ScFv) is typically formed by covalent bonding between the heavy-chain variable region and the light-chain variable region via a peptide linker, or by direct connection at the C-terminus, allowing it to form a dimeric structure similar to that of a two-chain Fv. Such antibody fragments can be obtained using proteolytic enzymes (for example, cleaving the whole antibody with papain yields Fab, and cleaving it with pepsin yields F(ab')2 fragments), or they can be produced using genetic recombination technology.

The antigen-binding fragment of the anti-CD19 antibody used in the present invention may be an scFv of the anti-CD19 antibody.

According to a specific embodiment, the antigen-binding fragment of the anti-CD19 antibody comprises a heavy chain variable region comprising an HCDR1 having the amino acid sequence selected from the group consisting of SEQ ID NO: 29, SEQ ID NO: 38 and SEQ ID NO: 47; an HCDR2 having the amino acid sequence region selected from the group consisting of SEQ ID NO: 30, SEQ ID NO: 39 and SEQ ID NO: 48; and an HCDR3 having the amino acid sequence selected from the group consisting of SEQ ID NO: 31, SEQ ID NO: 40 and SEQ ID NO: 49.

As used herein, the term "heavy chain" refers to both full-length heavy chain and fragments thereof comprising a variable region VH and three constant regions CH1, CH2, and CH3 with the amino acid sequence having sufficient variable region sequence to confer specificity to an antigen.

As used herein, the term "complementarity determining region (CDR)" refers to the amino acid sequence of the hypervariable region of the heavy and light chains of an immunoglobulin. The heavy chain (HCDR1, HCDR2, and HCDR3) and light chain (LCDR1, LCDR2, and LCDR3) each contain three CDRs, which provide the primary contact residues for an antibody to bind to an antigen or epitope.

The scope of the antibody or antigen-binding fragment of the present invention includes variants having conservative amino acid substitutions in the CDR region. Furthermore, the antibody or antigen-binding fragment of the present invention includes variants of the amino acid in the attached sequence listings, to the extent that they are capable of specifically recognizing NKp46. For example, additional changes can be made to the amino acid sequence of the antibody to further improve the binding affinity and/or other biological properties of the antibody. Such modifications include, for example, deletions, insertions, and/or substitutions of amino acid sequence residues of the antibody. These amino acid modifications are made based on the relative similarity of the amino acid side chain substituents, e.g., hydrophobicity, hydrophilicity, charge, size, etc. By analyzing the size, shape, and type of amino acid side chain substituents, it can be seen that arginine, lysine, and histidine are all positively charged residues; alanine, glycine, and serine have similar sizes; and phenylalanine, tryptophan, and tyrosine have similar shapes. Therefore, based on these considerations, arginine, lysine, and histidine; alanine, glycine, and serine; and phenylalanine, tryptophan, and tyrosine are biologically functional equivalents.

According to a more specific embodiment, the heavy chain variable region of the antigen-binding fragment of the anti-CD19 antibody comprises the amino acid sequence selected from the group consisting of SEQ ID NO: 32, SEQ ID NO: 41, and SEQ ID NO: 50.

According to a specific embodiment, the antigen-binding fragment of the anti-CD19 antibody further comprises a light chain variable region comprising an LCDR1 having the amino acid sequence selected from the group consisting of SEQ ID NO: 33, SEQ ID NO: 42 and SEQ ID NO: 51; an LCDR2 having the amino acid sequence region selected from the group consisting of SEQ ID NO: 34, SEQ ID NO: 43 and SEQ ID NO: 52; and an LCDR3 having the amino acid sequence selected from the group consisting of SEQ ID NO: 35, SEQ ID NO: 44 and SEQ ID NO: 53.

As used herein, the term "light chain" refers to both full-length light chain and fragments thereof comprising a variable region VL and a constant region CL with the amino acid sequence having sufficient variable region sequence to confer specificity to an antigen.

According to a specific embodiment, the light chain variable region of the antigen-binding fragment of the anti-CD19 antibody comprises the amino acid sequence selected from the group consisting of SEQ ID NO: 36, SEQ ID NO: 45, and SEQ ID NO: 54.

According to a specific embodiment, the antigen-binding fragment of the anti-CD19 antibody is any one selected from the group consisting of (a) to (c) below:
(a) an antigen-binding fragment comprising a heavy chain variable region comprising HCDR1 having the amino acid sequence of SEQ ID NO: 29, HCDR2 having the amino acid sequence of SEQ ID NO: 30, and HCDR3 having the amino acid sequence of SEQ ID NO: 31; and a light chain variable region comprising LCDR1 having the amino acid sequence of SEQ ID NO: 33, LCDR2 having the amino acid sequence of SEQ ID NO: 34, and LCDR3 having the amino acid sequence of SEQ ID NO: 35;
(b) an antigen-binding fragment comprising a heavy chain variable region comprising HCDR1 having the amino acid sequence of SEQ ID NO: 38, HCDR2 having the amino acid sequence of SEQ ID NO: 39, and HCDR3 having the amino acid sequence of SEQ ID NO: 40; and a light chain variable region comprising LCDR1 having the amino acid sequence of SEQ ID NO: 42, LCDR2 having the amino acid sequence of SEQ ID NO: 43, and LCDR3 having the amino acid sequence of SEQ ID NO: 44; and
(c) an antigen-binding fragment comprising a heavy chain variable region comprising HCDR1 having the amino acid sequence of SEQ ID NO: 47, HCDR2 having the amino acid sequence of SEQ ID NO: 48, and HCDR3 having the amino acid sequence of SEQ ID NO: 49; and a light chain variable region comprising LCDR1 having the amino acid sequence of SEQ ID NO: 51, LCDR2 having the amino acid sequence of SEQ ID NO: 52, and LCDR3 having the amino acid sequence of SEQ ID NO: 53.

More specifically, the antigen-binding fragment (a) comprises the heavy chain variable region having the amino acid sequence of SEQ ID NO: 32 and the light chain variable region having the amino acid sequence of SEQ ID NO: 36. Most specifically, the antigen-binding fragment (a) may be the anti-CD19 ScFv used in ProMab^{®} anti-CD19 CAR (Cat.# PM-CAR1001).

More specifically, the antigen-binding fragment (b) comprises the heavy chain variable region having the amino acid sequence of SEQ ID NO: 41 and the light chain variable region having the amino acid sequence of SEQ ID NO: 45. Most specifically, the antigen-binding fragment (a) may be the anti-CD19 ScFv used in Kymriah^{®} anti-CD19 CAR.

More specifically, the antigen-binding fragment (c) comprises the heavy chain variable region having the amino acid sequence of SEQ ID NO: 50 and the light chain variable region having the amino acid sequence of SEQ ID NO: 54. Most specifically, the antigen-binding fragment (c) may be the anti-CD19 ScFv used in Yescarta^{®} anti-CD19 CAR.

The antigen-binding fragment of the anti-BCMA antibody used in the present invention may be an scFv of the anti-BCMA antibody.

According to a specific embodiment, the antigen-binding fragment of the anti-BCMA antibody comprises a heavy chain variable region comprising an HCDR1 having the amino acid sequence of SEQ ID NO: 59; an HCDR2 having the amino acid sequence region of SEQ ID NO: 60; and an HCDR3 having the amino acid sequence of SEQ ID NO: 61.

According to another specific embodiment, the antigen-binding fragment of the anti-BCMA antibody comprises a heavy chain variable region comprising the amino acid sequence selected from the group consisting of SEQ ID NO: 56, SEQ ID NO: 62 and SEQ ID NO: 68.

According to a specific embodiment, the antigen-binding fragment of the anti-BCMA antibody further comprises a light chain variable region comprising an LCDR1 having the amino acid sequence selected of SEQ ID NO: 63; an LCDR2 having the amino acid sequence of SEQ ID NO: 64; and an LCDR3 having the amino acid sequence of SEQ ID NO: 65.

According to another specific embodiment, the antigen-binding fragment of the anti-BCMA antibody comprises a light chain variable region comprising the amino acid sequence selected from the group consisting of SEQ ID NO: 57, SEQ ID NO: 66 and SEQ ID NO: 69.

According to a specific embodiment, the antigen-binding fragment of the anti-BCMA antibody is any one selected from the group consisting of (d) to (f) below:
(d) an antigen-binding fragment comprising a heavy chain variable region having the amino acid sequence of SEQ ID NO: 56 and the light chain variable region having the amino acid sequence of SEQ ID NO: 57;
(e) an antigen-binding fragment comprising a heavy chain variable region comprising HCDR1 having the amino acid sequence of SEQ ID NO: 59, HCDR2 having the amino acid sequence of SEQ ID NO: 60, and HCDR3 having the amino acid sequence of SEQ ID NO: 61; and an antigen-binding fragment comprising the light chain variable region comprising LCDR1 having the amino acid sequence of SEQ ID NO: 63, LCDR2 having the amino acid sequence of SEQ ID NO: 64, and LCDR3 having the amino acid sequence of SEQ ID NO: 65; and
(f) an antigen-binding fragment comprising the heavy chain variable region having the amino acid sequence of SEQ ID NO: 68; and the light chain variable region having the amino acid sequence of SEQ ID NO: 69.

Most specifically, the antigen-binding fragment (d) may be the anti- BCMA ScFv used in ProMab^{®} anti- BCMA CAR (Cat.# PM-CAR1031).

More specifically, the antigen-binding fragment (e) comprises the heavy chain variable region having the amino acid sequence of SEQ ID NO: 62 and the light chain variable region having the amino acid sequence of SEQ ID NO: 66. Most specifically, the antigen-binding fragment (e) may be the anti-BCMA ScFv used in Abecma^{®} anti-BCMA CAR.

More specifically, the antigen-binding fragment (f) may be the anti-BCMA ScFv used in ProMab^{®} anti-BCMA CAR (Cat.# PM-CAR1037).

The immune cell of the present invention is introduced with a nucleic acid molecule encoding the aforementioned chimeric antigen receptor of the present invention and express it.

As used herein, the term "nucleic acid molecule" is meant to encompass DNA (gDNA and cDNA) and RNA molecules. Nucleotides, which are the basic structural units in nucleic acid molecules, include not only natural nucleotides, but also analogues having modified sugar or base moieties (Scheit, Nucleotide Analogs, John Wiley, New York (1980); Uhlman and Peyman, Chemical Reviews, 90:543-584 (1990)). The nucleic acid molecules of the present invention may be inserted into a gene delivery vector and introduced into target cells, such as immune cells, to express the aforementioned chimeric antigen receptor of the present invention.

As used herein, the term "express" refers to being artificially replicated as an extrachromosomal factor or by chromosomal integration in a target cell via a gene delivery system to cause the target cell to express an exogenous gene or overexpress an endogenous gene. Accordingly, "express" may be used interchangeably with "transformation", "transfection", or "transduction".

The term "gene delivery system" or "gene delivery vehicle" as used herein refers to any means for delivering a gene into a cell, and the term "gene delivery" has the same meaning as intracellular transduction of the gene. At the cell or tissue level, gene delivery has the same meaning as spread of the gene. Thus, the gene delivery system of the present invention may be referred to as a gene transduction system or a gene spread system.

To prepare the gene delivery system of the present invention, the nucleotide sequence of the present invention may be operably linked to a suitable expression regulatory sequence within a suitable expression construct. As used herein, the term "operatively linked" refers to a functional linkage between a nucleic acid expression regulatory sequence (e.g., a promoter, a signal sequence, or an array of transcription regulation factor binding sites) and the target nucleic acid sequence. Through the linkage, the regulatory sequence regulates the transcription and/or translation of the target nucleic acid sequence. A promoter linked to the target gene of the present invention is one capable of functioning in animal cells, more specifically in mammalian cells, and most specifically in immune cells, to regulate the transcription of the target gene. The promoter includes, but is not limited to, promoters derived from mammalian viruses and promoters derived from the genome of mammalian cells, such as the cytomegalovirus (CMV) promoter, the adenovirus late promoter, the vaccinia virus 7.5K promoter, the SV40 promoter, the HSV *tk* promoter, the RSV promoter, the EF1 alpha promoter, the metallothionein promoter, the beta-actin promoter, the promoter of the human IL-2 gene, the promoter of the human IFN gene, the promoter of the human IL-4 gene, the promoter of the human lymphotoxin gene, and the promoter of the human GM-CSF gene.

The nucleotide sequence of the target gene can be applied to any gene delivery system commonly used for gene introduction, such as plasmids, adenoviruses, adeno-associated viruses, retroviruses, lentiviruses, herpes simplex viruses, vaccinia viruses, liposomes, or niosomes.

As used herein, the term "immune cell" refers to any cell involved in the initiation or promotion of an immune response, and more specifically, an immune effector cell. Immune cells include, for example, but are not limited to, T cells, B cells, natural killer (NK) cells, natural killer T (NKT) cells, and mast cells. More specifically, the immune cells used in the present invention are selected from the group consisting of T cells, natural killer cells, and natural killer T (NKT) cells, and most specifically, T cells.

As used herein, the term "treating" refers to (a) inhibiting the progress of a disorder, disease or symptom; (b) alleviating the disorder, disease or symptom; or (c) eliminating the disorder, disease or symptom. The fusion protein of the present invention and CAR-expressing immune cells, when administered to a subject, induce the death of CD19-positive cancer cells, thereby inhibiting, eliminating, or alleviating the progression of symptoms caused by various CD19-positive tumors, including lymphoma. Accordingly, the term "treatment" or "therapeutic agent" in the present specification includes the meaning of "therapeutic aid" or "therapeutic adjuvant".

As used herein, the term "administer" or "administration" refers to the direct administration of a therapeutically effective amount of the composition of the present invention to a subject so that an equal amount is formed in the body of the subject.

As used herein, the terms "therapeutically effective amount" refer to the content of the composition of the present invention that is sufficient to provide a therapeutic or prophylactic effect to a subject to whom the composition is to be administered, and thus include the meaning of a "prophylactically effective amount".

As used herein, the term "subject" includes, without limitation, humans, mice, rats, guinea pigs, dogs, cats, horses, cows, pigs, monkeys, chimpanzees, baboons or rhesus monkeys. Specifically, the subject of the present invention is humans.

According to a specific embodiment, the cancer to be prevented or treated with the composition of the present invention is CD19-positive cancer. "CD19-positive cancer" refers to a solid tumor or hematological malignancy in which CD19 is overexpressed to a measurable degree compared to normal cells or CD19-negative tumors.

CD19 is a cell surface receptor expressed on the surface of B cells, such as pre-B cells, immature B cells in early stages of development, mature B cells, and malignant B cells. Since most malignant tumors of the B-cell lineage express CD19, active research is underway to develop it as a therapeutic target.

More specifically, the CD19-positive cancer is B-cell malignancy.

The B-cell malignancy includes, for example, chronic lymphocytic leukemia (CLL), acute lymphocytic leukemia (ALL), prolymphocytic leukemia, hairy cell leukemia, common acute lymphocytic leukemia (CALLA), non-Hodgkin lymphoma, diffuse large B-cell lymphoma (DLBCL), follicular lymphoma, splenic lymphoma, marginal zone lymphoma, mantle cell lymphoma, indolent B-cell lymphoma and Hodgkin lymphoma, but not limited thereto.

According to a specific embodiment, the cancer to be prevented or treated with the composition of the present invention is BCMA-positive cancer. "BCMA-positive cancer" refers to solid or hematologic cancers in which BCMA is overexpressed to a measurable degree compared to normal cells or BCMA-negative tumors.

BCMA is encoded by the TNFRSF17 gene and is a cell surface receptor of the TNF receptor superfamily that is primarily expressed on the surface of mature B cells and recognizes B-cell activating factors. It is particularly highly expressed in multiple myeloma and is widely studied as a diagnostic and therapeutic target.

More specifically, the BCMA-positive cancer is multiple myeloma.

In another aspect of this invention, there is provided an anticancer composition for co-administration with an immune cell that expresses a chimeric antigen receptor, comprising as an active ingredient a fusion protein comprising a CD80 protein or a fragment thereof and an IL-2 protein or a variant thereof.

Since the fusion protein and the CAR-expressing immune cell used in the present invention have already been described above in detail, descriptions thereof are omitted to avoid excessive redundancy.

When administered in combination with CAR-expressing immune cells, the fusion protein of the present invention maximizes anticancer efficacy and improves patient survival by enhancing and sustaining the immune cells' cancer cell killing activity. Co-administration may be performed by administering a single formulation comprising both the fusion protein of the present invention and CAR-expressing immune cells, or by simultaneously administering separate formulations comprising each agent individually, or by sequentially administering them in any order with an appropriate time interval.

In still another aspect of this invention, there is provided a pharmaceutical composition for preventing or treating cancer, comprising as active ingredients a fusion protein comprising a CD80 protein or a fragment thereof and an IL-2 protein or a variant thereof; and an immune cell that expresses a chimeric antigen receptor.

In still another aspect of this invention, there is provided a method for preventing or treating cancer, comprising administering to a subject in need thereof, a fusion protein comprising a CD80 protein or a fragment thereof and an IL-2 protein or a variant thereof; and an immune cell that expresses a chimeric antigen receptor.

Since the fusion protein and the CAR-expressing immune cell used in the present invention have already been described above in detail, descriptions thereof are omitted to avoid excessive redundancy.

### Advantageous Effects

The features and advantages of the present invention are summarized as follows:
(a) The present invention provides a composition for enhancing the activity of an immune cell that expresses a chimeric antigen receptor, comprising as an active ingredient, a fusion protein comprising a CD80 protein and an IL-2 protein
(b) When the fusion protein of the present invention is co-administered with chimeric antigen receptor (CAR)-expressing immune cells, specifically CAR-T cells, not only is the immune activity of the CAR-T cells within the tumor enhanced and sustained, but when the fusion protein of the present invention is administered alone to subjects in whom cancer has recurred after CAR-T cell administration, it can eliminate recurrent cancer cells by inducing the reactivation of previously administered CAR-T cells, thereby eliminating the recurrent cancer cells. Furthermore, even when cancer cells are re-implanted into an animal model in which cancer cells were completely eliminated by combined treatment of CAR-T cells and the fusion protein of the present invention, the immune activity of the CAR-T cells is maintained for a long period by the fusion protein, continuously suppressing the proliferation of the re-implanted cancer cells.
(c) Thus, the fusion protein of the present invention not only enhances and prolongs the anti-tumor immune activity of CAR-T cells but also maximizes the efficiency of CAR-T cells as a therapeutic cell resource by repeatedly reactivating previously administered CAR-T cells, thereby significantly improving survival rates of the patient.

### Brief Description of Drawings

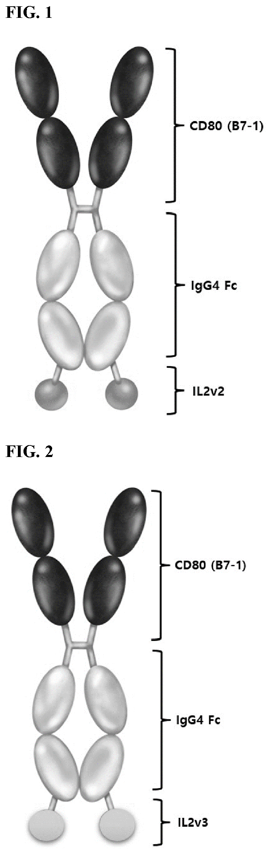

FIG. 1 schematically illustrates the structure of an example of the fusion protein (GI-101A).
FIG. 2 schematically illustrates the structure of an example of the fusion protein (GI-102).
FIG. 3 is a graph showing the number of dead Daudi cancer cells after co-culturing Daudi cancer cells (a type of B-cell lymphoma) and non-transduced T cells or CAR-T cells, with treating culture medium (Non-treat), IgG4-Fc and GI-101A, respectively followed by 16-hour incubation.
FIG. 4 represents a graph showing PD-1 expression in non-transduced T cells and CAR-T cells after co-culturing with Daudi cancer cells (a type of B-cell lymphoma) and treating the culture medium with either "Non-treat," IgG4-Fc, or GI-101A, followed by a 48-hour incubation.
FIG. 5 is the results of measuring dead cancer cells after co-culturing multiple myeloma U266 cells with non-transduced T cells or CAR-T cells in the presence of culture medium (Non-treat), IgG4-Fc, GI-101A, and GI-102, respectively, followed by a 16-hour incubation.
FIG. 6 is the results of measuring dead cancer cells after co-culturing multiple myeloma MM.1S cells with non-transduced T cells or CAR-T cells, treating them with culture medium (Non-treat), IgG4-Fc, GI-101A, and GI-102, respectively, followed by a 16-hour incubation.

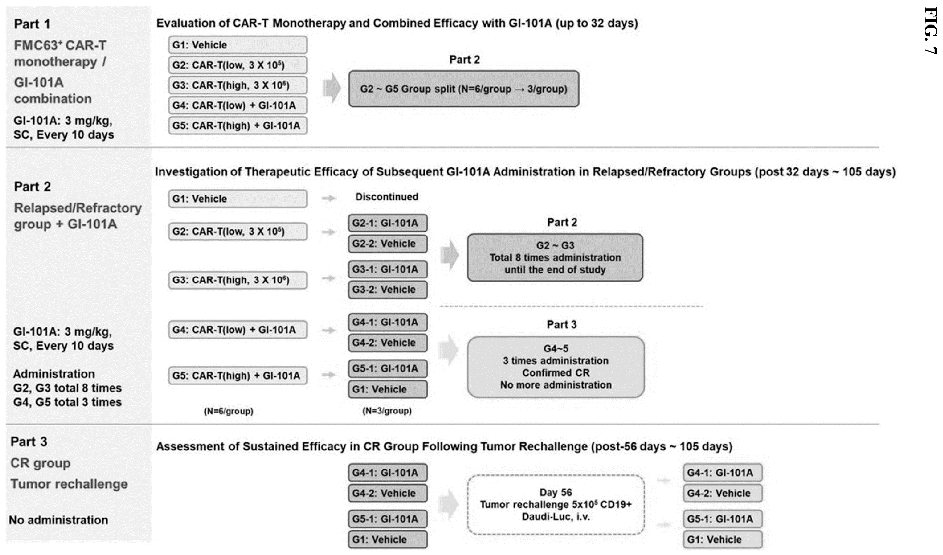

FIG. 7 is a diagram illustrating the experimental design to verify the combined effect of GI-101A and CAR-T cells using a lymphoma xenograft mouse model.
FIG. 8 shows the results of body weight measurements in the mouse model during Part 1 of the experiment described in Example 4.2.
FIG. 9 shows the images of tumor progression captured using an IVIS (In-Vivo Imaging System) at days 0, 7, 20, and 32 after administration in a lymphoma xenograft mouse model of IgG4-Fc alone (Vehicle, G1); low-dose (3 × 10⁵ cells) CAR-T cells in combination with IgG4-Fc [CAR-T (low), G2]; high-dose (3 × 10⁶ cells) CAR-T cells in combination with IgG4-Fc [CAR-T (high), G3]; low-dose CAR-T cells in combination with GI-101A (G4); and high-dose CAR-T cells in combination with GI-101A (G5).
FIG. 10 is a graph showing the total light emission from the images captured in FIG. 9.
FIG. 11 shows the results of body weight measurements in the mouse model during Part 2 of the experiment described in Example 4.3.
FIG. 12 shows IVIS images of tumor progression captured on days 42, 53, 62, 74, 84, 95, and 104 after dividing the groups into those receiving low-dose (3 × 10⁵ cells) CAR-T cells in combination with IgG4-Fc [CAR-T (low), G2] and high-dose (3 × 10⁶ cells) CAR-T cells in combination with IgG4-Fc [CAR-T (high), G3], followed by administration of either IgG4-Fc or GI-101A, respectively.
FIG. 13 is a graph showing the total light emission from the images captured in FIG. 12.
FIG. 14 shows the results of body weight measurements in the mouse model during Part 3 of the experiment described in Example 4.4.
FIG. 15 shows IVIS images of tumor progression in groups administered low-dose (3 × 10⁵ cells) CAR-T cells in combination with GI-101A (G4) and high-dose (3 × 10⁶ cells) CAR-T cells in combination with GI-101A (G5). After dividing these groups, IgG4-Fc or GI-101A was administered, and tumor progression was imaged on days 42 and 53. Tumor cells were then re-inoculated on day 56, and subsequent tumor progression was imaged on days 62, 74, 84, 95, and 104 using an IVIS system.
FIG. 16 is a graph showing the total light emission from the results captured in FIG. 15.
FIG. 17 shows the number of FMC63⁺CD3⁺T cells, CD3⁺CD4⁺T cells and CD3⁺CD8⁺T cells on days 1, 11, 21, and 33 in Part 1 of the experiment described in Example 4.2.
FIG. 18 shows the number of FMC63⁺CD3⁺T cells, CD3⁺CD4⁺T cells and CD3⁺CD8⁺T cells on days 33, 44, 54, 63, 75, 85, 95 and 105 in Part 2 of the experiment described in Example 4.3.
FIG. 19 shows the number of FMC63⁺CD3⁺T cells, CD3⁺CD4⁺T cells and CD3⁺CD8⁺T cells on days 33, 44, 54, 63, 75, 85, 95 and 105 in Part 3 of the experiment described in Example 4.4.

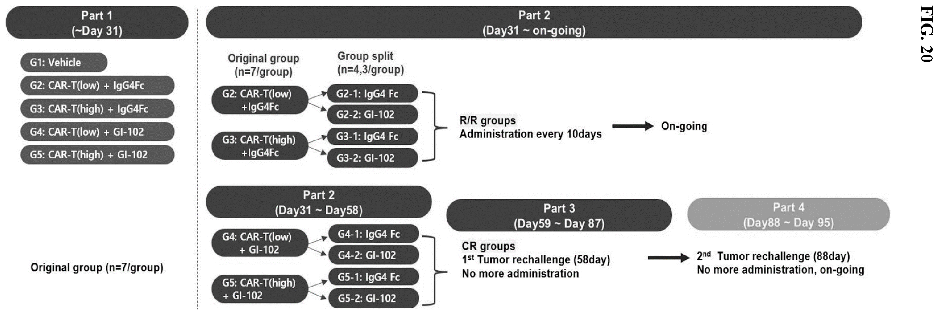

FIG. 20 is a diagram illustrating the experimental design to verify the combined effect of GI-102 and CAR-T cells using a lymphoma xenograft mouse model.
FIG. 21 shows IVIS images of tumor progression in a lymphoma xenograft mouse model captured on days 0, 10, 20, and 30 after administration of IgG4-Fc alone (Vehicle, G1); low-dose (3 × 10⁵ cells) CAR-T cells in combination with IgG4-Fc [CAR-T (low), G2]; high-dose (3 × 10⁶ cells) CAR-T cells in combination with IgG4-Fc [CAR-T (high), G3]; low-dose CAR-T cells in combination with GI-102 (G4); and high-dose CAR-T cells in combination with GI-102 (G5).
FIG. 22 shows IVIS images of tumor progression captured on days 40, 50, 66, 77, 87, and 95 after dividing the groups into those receiving low-dose (3 × 10⁵ cells) CAR-T cells in combination with IgG4-Fc [CAR-T (low), G2] and high-dose (3 × 10⁶ cells) CAR-T cells in combination with IgG4-Fc [CAR-T (high), G3], followed by administration of either IgG4-Fc or GI-102, respectively.
FIG. 23 shows IVIS images of tumor progression in groups administered low-dose (3 × 10⁵ cells) CAR-T cells in combination with GI-101A (G4) and high-dose (3 × 10⁶ cells) CAR-T cells in combination with GI-102 (G5). After dividing these groups, IgG4-Fc or GI-102 was administered, and tumor progression was imaged on days 40 and 53. Tumor cells were then re-inoculated on day 59, and subsequent tumor progression was imaged on days 66, 77, 87, and 95 using an IVIS system.
FIG. 24 shows the number of CD3⁺CD4⁺T cells and CD3⁺CD8⁺T cells on day 11 in Part 1 of Example 6.2.
FIG. 25 shows the number of CD3⁺CD4⁺T cells and CD3⁺CD8⁺T cells on day 52 in Part 2 of Example 6.3.
FIG. 26 shows the number of CD3⁺CD4⁺T cells and CD3⁺CD8⁺T cells on day 52 in Part 3 of Example 6.4.
FIG. 27 is a graph showing the degree of tumor formation, expressed as total flux, measured on day 23 after administration of BCMA-targeted CAR-T cells and IgG4; and BCMA-targeted CAR-T cells and GI-102; to a multiple myeloma mouse model.
FIG. 28 is a graph showing the results of measuring the number of CD3⁺CD4⁺T cells and CD3⁺CD8⁺T cells in blood samples taken 24 days after administration of BCMA-targeted CAR-T cells and IgG4; and BCMA-targeted CAR-T cells and GI-102; to a multiple myeloma mouse model.

### Mode for Invention

Hereinafter, the present invention will be described in more detail by way of examples. These examples are only for illustrating the present invention in more detail, and it will be apparent to those skilled in the art that the scope of the present invention according to the subject matter of the present invention is not limited by these examples.

### Examples

### I. Preparation of the Fusion Protein

### Preparative Example 1. Preparation of GI-101A

To produce a fusion protein comprising a human CD80 fragment, an Fc domain and an IL-2 variant, a signal peptide (SEQ ID NO: 1), a CD80 fragment (SEQ ID NO: 2), an Ig hinge (SEQ ID NO: 3), an Fc domain (SEQ ID NO: 4), a linker (SEQ ID NO: 5), and an IL-2 variant (2M) (R38A, F42A) (SEQ ID NO: 6) in that order from the N-terminus, was synthesized via Thermo Fisher Scientific's Invitrogen GeneArt Gene Synthesis service and cloned into the pcDNA3.4 vector (Table 1).

**[Table 1]**

| Category | Sequence | SEQ ID NO |
|---|---|---|
| signal peptide (TPA) | MDAMLRGLCCVLLLCGAVFVSPSHA | 1 |
| CD80 (hB7-1:35-242) | | 2 |
| hinge | GSGGGGSGGGGSGGGGSAESKYGPPCPPCP | 3 |
| Immuno-globulin Fc | APEAAGGPSVFLFPPKPKDQLMISRTPEVTCVVVDVSQEDPEVQF NWYVDGVEVHNAKTKPREEQFNSTYRVVSVLTVLHQDWLNGKE YKCKVSNKGLPSSIEKTISKAKGQPREPQVYTLPPSQEEMTKNQVS LTCLVKGFYPSDIAVEWESNGQPENNYKTTPPVLDSDGSFFLYSR LTVDKSRWQEGNVFSCSVLHEALHNHYTQKSLSLSLG | 4 |
| linker | GGGGS | 5 |
| hIL2v2 | APTSSSTKKTQLQLEHLLLDLQMILNGINNYKNPKLTAMLTAKFY MPKKATELKHLQCLEEELKPLEEVLNLAQSKNFHLRPRDLISNINV IVLELKGSETTFMCEYADETATIVEFLNR WITFCQSIISTLT | 6 |
| CD80-IgG4 Fc-IL2v2 | ATGGATGCTATGCTGAGAGGCCTGTGTTGCGTGCTGCTGCTGT GTGGCGCTGTGTTCGTGTCTCCTTCTCACGCTGTGATCCACGTG ACCAAAGAAGTGAAAGAGGTCGCCACACTGTCCTGCGGCCAC AACGTTTCAGTGGAAGAACTGGCCCAGACCAGGATCTACTGGC AGAAAGAAAAGAAAATGGTGCTGACCATGATGTCCGGCGACA TGAACATCTGGCCTGAGTACAAGAACCGGACCATCTTCGACAT CACCAACAACCTGTCCATCGTGATTCTGGCCCTGAGGCCTTCTG ATGAGGGCACCTATGAGTGCGTGGTGCTGAAGTACGAGAAGG ACGCCTTCAAGCGCGAGCACCTGGCTGAAGTGACACTGTCCGT GAAGGCCGACTTTCCCACACCTTCCATCTCCGACTTCGAGATCC CTACCTCCAACATCCGGCGGATCATCTGTTCTACCTCTGGCGGC TTTCCTGAGCCTCACCTGTCTTGGCTGGAAAACGGCGAGGAAC TGAACGCCATCAACACCACCGTGTCTCAGGACCCCGAAACCGA GCTGTACGCTGTGTCCTCCAAGCTGGACTTCAACATGACCACC AACCACAGCTTCATGTGCCTGATTAAGTACGGCCACCTGAGAG TGAACCAGACCTTCAACTGGAACACCACCAAGCAAGAGCACTT CCCTGACAATGGATCTGGCGGCGGAGGTTCTGGCGGAGGTGGA AGCGGAGGCGGAGGATCTGCTGAGTCTAAGTATGGCCCTCCTT GTCCTCCATGTCCTGCTCCAGAAGCTGCTGGCGGACCCTCTGTG TTCCTGTTTCCTCCAAAGCCTAAGGACCAGCTCATGATCTCTCG GACACCCGAAGTGACCTGCGTGGTGGTGGATGTGTCTCAAGAG GACCCTGAGGTGCAGTTCAATTGGTACGTGGACGGCGTGGAAG TGCACAACGCCAAGACCAAGCCTAGAGAGGAACAGTTCAACT CCACCTACAGAGTGGTGTCCGTGCTGACCGTGCTGCACCAGGA TTGGCTGAACGGCAAAGAGTACAAGTGCAAGGTGTCCAACAA GGGCCTGCCTTCCAGCATCGAAAAGACCATCTCCAAGGCTAAG GGCCAGCCTAGGGAACCCCAGGTTTACACCCTGCCTCCAAGCC AAGAGGAAATGACCAAGAACCAGGTGTCCCTGACCTGCCTGGT CAAGGGCTTCTACCCTTCCGACATTGCCGTGGAATGGGAGTCC AATGGCCAGCCTGAGAACAACTACAAGACCACACCTCCTGTGC TGGACTCCGACGGCTCCTTCTTTCTGTACTCTCGCCTGACCGTG GACAAGTCTAGATGGCAAGAGGGCAACGTGTTCTCCTGCTCTG TGCTGCACGAGGCCCTGCACAATCACTACACCCAGAAGTCCCT GTCTCTGTCTCTTGGAGGTGGTGGCGGTTCTGCCCCTACCAGCT CCTCTACCAAGAAAACCCAGCTCCAGTTGGAGCATCTGCTGCT GGACCTCCAGATGATTCTGAACGGGATCAACAACTATAAGAAC CCCAAGCTGACCGCCATGCTGACCGCTAAGTTCTACATGCCCA AGAAGGCCACCGAGCTGAAGCACCTCCAGTGCCTGGAAGAAG AACTGAAGCCCCTGGAAGAGGTGCTGAATCTGGCCCAGTCCAA GAACTTCCACCTGAGGCCACGGGACCTGATCAGCAACATCAAC GTGATCGTGCTGGAACTGAAGGGCTCCGAGACAACCTTTATGT GCGAGTACGCCGACGAGACAGCCACCATCGTGGAATTTCTGAA CCGGTGGATCACCTTCTGCCAGAGCATCATCTCCACACTGACC TGATGA | 8 |
| CD80-IgG4 Fc-IL2v2 | VIHVTKEVKEVATLSCGHNVSVEELAQTRIYWQKEKKMVLTMM SGDMNIWPEYKNRTIFDITNNLSIVILALRPSDEGTYECVVLKYEK DAFKREHLAEVTLSVKADFPTPSISDFEIPTSNIRRIICSTSGGFPEPH LSWLENGEELNAINTTVSQDPETELYAVSSKLDFNMTTNHSFMCL IKYGHLRVNQTFNWNTTKQEHFPDNGSGGGGSGGGGSGGGGSA ESKYGPPCPPCPAPEAAGGPSVFLFPPKPKDQLMISRTPEVTCVVV DVSQEDPEVQFNWYVDGVEVHNAKTKPREEQFNSTYRVVSVLT VLHQDWLNGKEYKCKVSNKGLPSSIEKTISKAKGQPREPQVYTLP PSQEEMTKNQVSLTCLVKGFYPSDIAVEWESNGQPENNYKTTPPV LDSDGSFFLYSRLTVDKSRWQEGNVFSCSVLHEALHNHYTQKSLS LSLGGGGGSAPTSSSTKKTQLQLEHLLLDLQMILNGINNYKNPKL TAMLTAKFYMPKKATELKHLQCLEEELKPLEEVLNLAQSKNFHL RPRDLISNINVIVLELKGSETTFMCEYADETATIVEFLNRWITFCQS IISTLT | 9 |

Furthermore, the vector was introduced into CHO cells (Expi-CHO) to express the fusion protein of SEQ ID NO: 9. In a stable cell line expressing the fusion protein of SEQ ID NO: 9, feed medium was supplied every other day at a rate of 7.0% (v/v) from day 3 to day 7 of culture using a fed-batch culture method, and the culture supernatant was harvested on day 7. The fusion protein (GI-101A) was purified from the recovered culture supernatant via chromatography.

### Preparative Example 2. Preparation of GI-102

To produce a fusion protein comprising a human CD80 fragment, an Fc domain, and an IL-2 variant (3M) with three amino acid substitutions (R38A, F42A, E61R) (GI101-M61), the signal peptide (SEQ ID NO: 1), a CD80 fragment (SEQ ID NO: 2), an Ig hinge (SEQ ID NO: 3), an Fc domain (SEQ ID NO: 4), a linker (SEQ ID NO: 5), and an IL-2 variant (SEQ ID NO: 23), was synthesized via Thermo Fisher Scientific's Invitrogen GeneArt Gene Synthesis service and cloned into the pcDNA3.4 vector (Table 2).

**[Table 2]**

| Category | Sequence Information | SEQ NO |
|---|---|---|
| Signal peptide (TPA) | MDAMLRGLCCVLLLCGAVFVSPSHA | 1 |
| CD80 (hB7-1:35-242) | | 2 |
| hinge | GSGGGGSGGGGSGGGGSAESKYGPPCPPCP | 3 |
| Immunog lobulin- s in Fc | APEAAGGPSVFLFPPKPKDQLMISRTPEVTCVVVDVSQEDPEVQ FNWYVDGVEVHNAKTKPREEQFNSTYRVVSVLTVLHQDWLN GKEYKCKVSNKGLPSSIEKTISKAKGQPREPQVYTLPPSQEEM TKNQVSLTCLVKGFYPSDIAVEWESNGQPENNYKTTPPVLDSD GSFFLYSRLTVDKSRWQEGNVFSCSVLHEALHNHYTQKSLSLS LG | 4 |
| linker | GGGGS | 5 |
| hIL2v3 | APTSSSTKKTQLQLEHLLLDLQMILNGINNYKNPKLTAMLTAK FYMPKKATELKHLQCLERELKPLEEVLNLAQSKNFHLRPRDLIS NINVIVLELKGSETTFMCEYADETATIVEFLNRWITFCQSIISTLT | 23 |
| CD80-IgG4 Fc-IL2v3 | ATGGATGCTATGCTGAGAGGCCTGTGTTGCGTGCTGCTGCTG TGTGGCGCTGTGTTCGTGTCTCCTTCTCACGCTGTGATCCAC GTGACCAAAGAAGTGAAAGAGGTCGCCACACTGTCCTGCGG CCACAACGTTTCAGTGGAAGAACTGGCCCAGACCAGGATCT ACTGGCAGAAAGAAAAGAAAATGGTGCTGACCATGATGTCC GGCGACATGAACATCTGGCCTGAGTACAAGAACCGGACCAT CTTCGACATCACCAACAACCTGTCCATCGTGATTCTGGCCCT GAGGCCTTCTGATGAGGGCACCTATGAGTGCGTGGTGCTGA AGTACGAGAAGGACGCCTTCAAGCGCGAGCACCTGGCTGAA GTGACACTGTCCGTGAAGGCCGACTTTCCCACACCTTCCATC TCCGACTTCGAGATCCCTACCTCCAACATCCGGCGGATCATC TGTTCTACCTCTGGCGGCTTTCCTGAGCCTCACCTGTCTTGGC TGGAAAACGGCGAGGAACTGAACGCCATCAACACCACCGTG TCTCAGGACCCCGAAACCGAGCTGTACGCTGTGTCCTCCAAG CTGGACTTCAACATGACCACCAACCACAGCTTCATGTGCCTG ATTAAGTACGGCCACCTGAGAGTGAACCAGACCTTCAACTG GAACACCACCAAGCAAGAGCACTTCCCTGACAATGGATCTG GCGGCGGAGGTTCTGGCGGAGGTGGAAGCGGAGGCGGAGG ATCTGCTGAGTCTAAGTATGGCCCTCCTTGTCCTCCATGTCCT GCTCCAGAAGCTGCTGGCGGACCCTCTGTGTTCCTGTTTCCT CCAAAGCCTAAGGACCAGCTCATGATCTCTCGGACACCCGA AGTGACCTGCGTGGTGGTGGATGTGTCTCAAGAGGACCCTG AGGTGCAGTTCAATTGGTACGTGGACGGCGTGGAAGTGCAC AACGCCAAGACCAAGCCTAGAGAGGAACAGTTCAACTCCAC CTACAGAGTGGTGTCCGTGCTGACCGTGCTGCACCAGGATTG GCTGAACGGCAAAGAGTACAAGTGCAAGGTGTCCAACAAGG GCCTGCCTTCCAGCATCGAAAAGACCATCTCCAAGGCTAAG GGCCAGCCTAGGGAACCCCAGGTTTACACCCTGCCTCCAAG CCAAGAGGAAATGACCAAGAACCAGGTGTCCCTGACCTGCC TGGTCAAGGGCTTCTACCCTTCCGACATTGCCGTGGAATGGG AGTCCAATGGCCAGCCTGAGAACAACTACAAGACCACACCT CCTGTGCTGGACTCCGACGGCTCCTTCTTTCTGTACTCTCGCC TGACCGTGGACAAGTCTAGATGGCAAGAGGGCAACGTGTTC TCCTGCTCTGTGCTGCACGAGGCCCTGCACAATCACTACACC CAGAAGTCCCTGTCTCTGTCTCTTGGAGGTGGTGGCGGTTCT GCCCCTACCAGCTCCTCTACCAAGAAAACCCAGCTCCAGTTG GAGCATCTGCTGCTGGACCTCCAGATGATTCTGAACGGATC AACAACTATAAGAACCCCAAGCTGACCGCCATGCTGACCGC TAAGTTCTACATGCCCAAGAAGGCCACCGAGCTGAAGCACC TCCAGTGCCTGGAAAGGGAACTGAAGCCCCTGGAAGAGGTG CTGAATCTGGCCCAGTCCAAGAACTTCCACCTGAGGCCACG GGACCTGATCAGCAACATCAACGTGATCGTGCTGGAACTGA AGGGCTCCGAGACAACCTTTATGTGCGAGTACGCCGACGAG ACAGCCACCATCGTGGAATTTCTGAACCGGTGGATCACCTTC TGCCAGAGCATCATCTCCACACTGACC | 27 |
| CD80-IgG4 Fc-IL2v3 | VIHVTKEVKEVATLSCGHNVSVEELAQTRIYWQKEKKMVLTM MSGDMNIWPEYKNRTIFDITNNLSIVILALRPSDEGTYECVVLK YEKDAFKREHLAEVTLSVKADFPTPSISDFEIPTSNIRRIICSTSG GFPEPHLSWLENGEELNAINTTVSQDPETELYAVSSKLDFNMTT NHSFMCLIKYGHLRVNQTFNWNTTKQEHFPDNGSGGGGSGGG GSGGGGSAESKYGPPCPPCPAPEAAGGPSVFLFPPKPKDQLMIS RTPEVTCVVVDVSQEDPEVQFNWYVDGVEVHNAKTKPREEQF NSTYRVVSVLTVLHQDWLNGKEYKCKVSNKGLPSSIEKTISKA KGQPREPQVYTLPPSQEEMTKNQVSLTCLVKGFYPSDIAVEWE SNGQPENNYKTTPPVLDSDGSFFLYSRLTVDKSRWQEGNVFSC SVLHEALHNHYTQKSLSLSLGGGGGSAPTSSSTKKTQLQLEHLL LDLQMILNGINNYKNPKLTAMLTAKFYMPKKATELKHLQCLE RELKPLEEVLNLAQSKNFHLRPRDLISNINVIVLELKGSETTFMC EYADETATIVEFLNRWITFCQSIISTLT | 28 |

Furthermore, the vector was introduced into CHO cells (Expi-CHO) to express the fusion protein of SEQ ID NO: 28. The stable cell line expressing the fusion protein of SEQ ID NO: 28 was cultured in a fed-batch system, with feed medium supplied at a rate of 7.0% (v/v) every other day from day 3 to day 11, and the culture supernatant was harvested on day 12. The fusion protein (GI-102) was purified from the culture supernatant recovered via chromatography.

### II. Verification of the fusion protein's effect on enhancing the immune activity of CAR-T cell: in vitro

### Example 1. Enhancement of Cytotoxic Activity of CD19-Targeted CAR-T Cells by GI-101A

To distinguish the target cells (Daudi cancer cells, Biocytogen Pharmaceuticals, Cat. No. 310728) from the effector cells (T cells), Daudi cancer cells were labeled with the CTV (CellTrace Violet, Invitrogen) dye. Specifically, Daudi cancer cells into which luciferase cDNA had been inserted were labeled by incubating with 1 µM CTV dye at 37°C for 20 minutes. After the reaction was complete, a volume of culture medium five times that of the staining solution was added and reacted for 5 minutes, followed by centrifugation at 1,300 rpm for 5 minutes to remove unbound CTV dye from the cells. The CTV-labeled Daudi cancer cells were resuspended in culture medium [RPMI 1640 medium containing 10% fetal bovine serum (FBS), 100 U/mL penicillin/streptomycin, and 2 mM L-glutamine], and seeded into a 96-well microplate at 5 × 10⁴ cells per well.

T cells engineered with a chimeric antigen receptor (CAR) gene (CD19scFv-CD28-CD3ζ) (ProMab Biotechnologies, Cat. No. PM-CAR1001) and non-transduced T cells without the CAR gene (ProMab Biotechnologies, Cat. No. PM-CAR2003) were prepared as a control and dispensed at 5 × 10⁴ cells per well into a 96-well microplate containing Daudi cancer cells labeled with CTV dye (ET ratio = 1:1). Wells containing only target cells (control group) were filled with an equal volume of culture medium.

IgG4-Fc (50 nM) or GI-101A (50 nM) was added to each well of the 96-well microplates containing target and effector cells, and the plates were incubated for 16 hours at 37°C and 5% CO₂. The cells co-cultured for 16 hours were centrifuged to remove the supernatant, resuspended in FACS buffer [DPBS buffer containing 2% fetal bovine serum (FBS)], and centrifuged again. Subsequently, Annexin V binding buffer (Biolegend, Cat. No. 422201), 7-AAD (Biolegend, Cat. No. 420403) and FITC Annexin V (Biolegend, Cat. No. 640906) were added, and the mixture was incubated at 4°C for 20 minutes. After the reaction was complete, the samples were analyzed using a Cytek Aurora (Cytek Biosciences) flow cytometer and FlowJo Software (BD Biosciences).

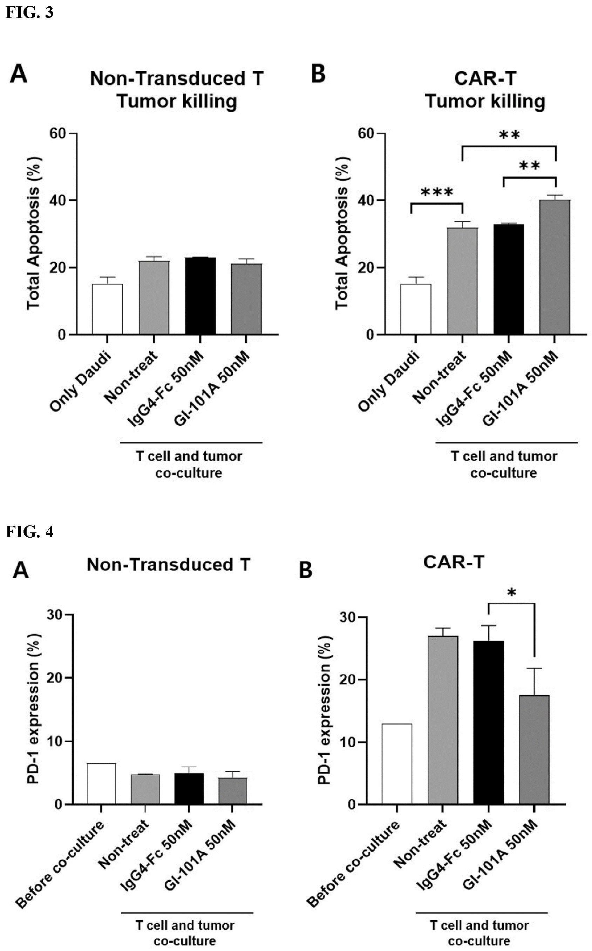

As results, non-transduced T cells exhibited minimal tumor-killing activity against Daudi cancer cells, and treatment with GI-101A did not significantly alter this activity. In contrast, CAR-T cells demonstrated tumor-killing activity against Daudi cancer cells, and treatment with GI-101A increased the tumor-killing activity of CAR-T cells (FIG. 3).

### Example 2. PD-1 expression in CAR-T cells was reduced by GI-101A

Daudi cancer cells were labeled with CTV dye using the same method as in Example 1, and resuspended in culture medium [RPMI 1640 medium containing 10% fetal bovine serum (FBS), 100 U/mL penicillin/streptomycin, and 2 mM L-glutamine], and then seeded into a 96-well microplate at 5 × 10⁴ cells per well.

CAR-engineered T cells and non-transduced T cells (as a control) were prepared separately and dispensed at 5 × 10⁴ cells per well into a 96-well microplate containing Daudi cancer cells labeled with CTV dye (ET ratio = 1:1). Wells containing only target cells (control group) were filled with the same volume of culture medium.

IgG4-Fc (50 nM) or GI-101A (50 nM) was added to the 96-well microplate each containing target and effector cells, and the plates were incubated for 48 at 37°C and 5% CO₂. The cultured cells were centrifuged to remove the supernatant, resuspended in FACS buffer [DPBS buffer containing 2% fetal bovine serum (FBS)], and centrifuged again. After centrifugation, the cells were resuspended in FACS buffer, treated with Fc blocker (BD Biosciences, Cat. No. 564219) and then incubated at 4°C for 5 minutes. After the reaction was completed, APC anti-FMC63 (ACRO Biosystems, FM3-AY54A1-25tests), Fixable Viability Dye eFluor^{™} 780 (eBioscience, Cat. No. 65-0865-14), PE-Cy7 anti-CD3 (Biolegend, Cat. No. 300420) and BV510 anti-PD-1 (BD Biosciences, Cat. No. 563076) were treated and incubated at 4°C for 20 minutes before washing with FACS buffer. The cell pellets were resuspended in FACS buffer and analyzed using a Cytek Aurora flow cytometer and FlowJo software.

The results showed that while there was almost no change in PD-1 expression in non-transduced T cells before and after co-culture with Daudi cancer cells, PD-1 expression increased in CAR-T cells after co-culture with Daudi cancer cells. Furthermore, while there was no significant change in PD-1 expression by co-culture in CAR-T cells treated with IgG4-Fc, PD-1 expression was significantly reduced by co-culture in CAR-T cells treated with GI-101A (FIG. 4).

It is known that reduced PD-1 expression in CAR-T cells enhances their tumor cell-killing ability. In other words, as PD-1 expression decreases, CAR-T cell infiltration into tumor cells increases, thereby suppressing tumor formation and potentially improving the therapeutic response.

FIG. 4 confirms that GI-101A enhances the effector function of CAR-T cells, which destroys tumor cells and induces an immune response.

### Example 3. Enhancement of the cytolytic activity of BCMA-targeted CAR-T cells by GI-101A or GI-102

To distinguish between the target cells U266 (human multiple myeloma cells) or MM.1S (human multiple myeloma B lymphoblasts) and the effector T cells, the multiple myeloma cells were labeled with the CTV (CellTrace^{™} Violet, Invitrogen^{™}) dye. Briefly, Daudi cancer cells into which luciferase cDNA had been inserted were labeled by incubating with 1 µM CTV dye at 37°C for 20 minutes. After the reaction was completed, a volume of culture medium five times that of the staining solution was added and allowed to react for 5 minutes, followed by centrifugation at 1,300 rpm for 5 minutes to remove unbound CTV dye from the cells. The CTV-labeled multiple myeloma cells were resuspended in culture medium [RPMI 1640 medium containing 10% fetal bovine serum (FBS), 100 U/mL penicillin/streptomycin, and 2 mM L-glutamine], and then seeded into a 96-well microplate at 5 × 10⁴ cells per well.

T cells expressing the CAR gene (BCMAscFv-CD28-CD3ζ) (ProMab Biotechnologies, Cat. No. PM-CAR1031) and, as a control, non-transduced T cells without the CAR gene (ProMab Biotechnologies, Cat. No. PM-CAR2003) were prepared and seeded at 5 × 10⁴ cells per well into a 96-well microplate containing Daudi cancer cells labeled with CTV dye (ET ratio = 1:1). Wells containing only target cells (controls) were filled with an equal volume of culture medium.

IgG4-Fc (50 nM), GI-101A (50 nM) or GI-102 (50 nM) was added to each well of the 96-well microplates containing target and effector cells, and the plates were incubated for 16 hours at 37°C and 5% CO₂. The cells co-cultured for 16 hours were centrifuged to remove the supernatant, resuspended in FACS buffer [DPBS buffer containing 2% fetal bovine serum (FBS)], and centrifuged again. Subsequently, Annexin V binding buffer (Biolegend, Cat. No. 422201), 7-AAD (Biolegend, Cat. No. 420403) and FITC Annexin V (Biolegend, Cat. No. 640906) were added, and the mixture was incubated at 4°C for 20 minutes. After the reaction was completed, the samples were analyzed using a Cytek Aurora (Cytek Biosciences) flow cytometer and FlowJo Software (BD Biosciences).

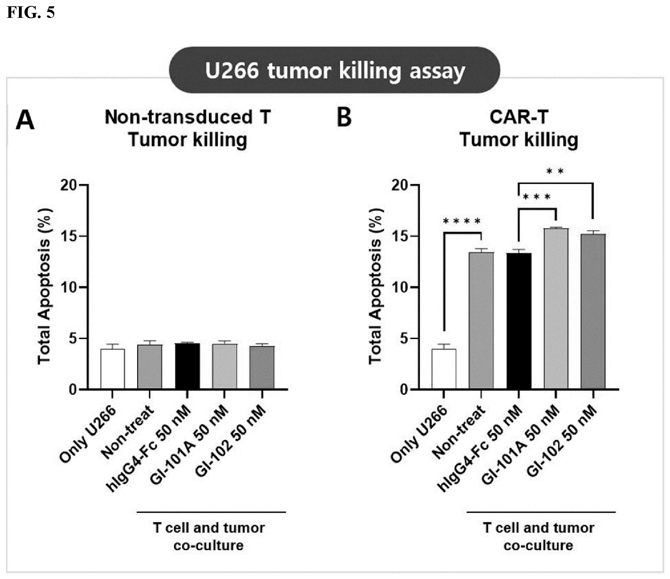

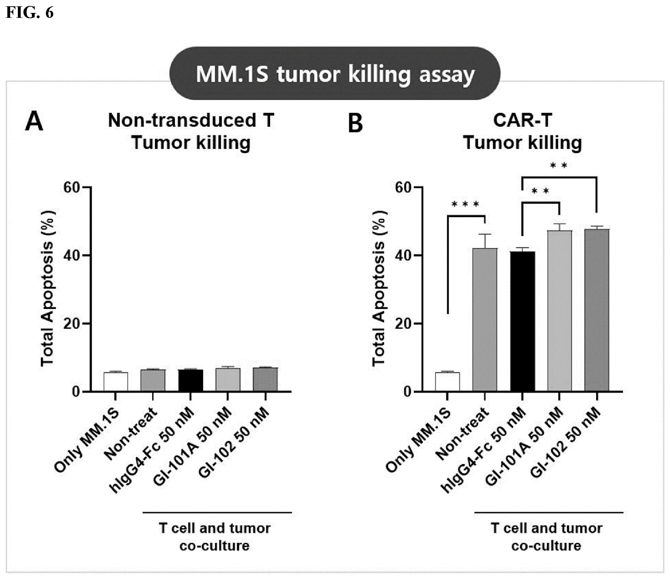

As results, non-transduced T cells exhibited minimal cytotoxic activity against U266 and MM.S1 cancer cells, and treatment with GI-101A or GI-102 had little effect. In contrast, CAR-T cells exhibited cytotoxic activity against U266 and MM.S1 cancer cells, which was enhanced by GI-101A and GI-102 (FIGS. 5 and 6).

### III. The effect of fusion protein on immune activity of CAR-T cell: in vivo

### Example 4. The effects of GI-101A on the reactivation of CD19-targeted CAR-T cells and the enhancement of cytotoxic activity against cancer cells in a lymphoma xenograft mouse model

### Example 4.1. Establishment of a lymphoma NSG mouse model and isolation of experimental groups

To establish a lymphoma xenograft mouse model, 5×10⁵ B-Luc Daudi cancer cells expressing luciferase were injected into the tail vein of 4-8-week-old NSG mice (NOD scid gamma mouse, Jackson Labs) at a dose of 200 µL per mouse. Following B-Luc Daudi cell administration, *in vivo* imaging was performed 7 days prior to CAR-T cell administration. Bioluminescence intensity comparable across groups was confirmed using the IVIS Lumina III (PerkinElmer, Inc.), after which the mice were classified into each group. To measure bioluminescence, luciferin was administered intraperitoneally 10 minutes prior to *in vivo* imaging. Animals were maintained under isoflurane-inhalation anesthesia during imaging. Following *in vivo* imaging, a region of interest (ROI) was designated for each subject, and photon counts within that region were recorded. To monitor the progress of the experiment, bioluminescence measurements were performed a total of 11 times on days 0, 7, 20, 32, 42, 53, 62, 74, 84, 95, and 104.

Thirty mice in good health were randomly selected based on the physiological condition, body weight changes and tumor growth rates. The selected animals were divided into groups of six per group, ensuring as much uniformity as possible based on tumor formation and body weight.

### Example 4.2. Administration of Test Substance and Tumor Growth Evaluation (Part 1)

### The test groups to be administered with the test substance were configured as shown in Table 3 below. The period from the CAR-T administration date (Day 0) to the test substance administration dates (Days 1 through 33) was defined as Part 1.

**[Table 3]**

| Group | Sex | Number of Subjects | Test Substance | Administration | | | |
|---|---|---|---|---|---|---|---|
| | | | | Dose | Volume (mL/kg) | Route | Frequency |
| G1 | F | 6 | IgG4 | 1.1 mg/kg | 5 | S.C. | Once every 10 days (Q10D) x 3 |
| G2 | F | 6 | CAR-T (low) | 3 × 10⁵cells/patient | 5 | I.V. | 1 dose |
| | | | IgG4 | 1.1 mg/kg | 5 | S.C. | Q10D x 3 |
| G3 | F | 6 | CAR-T (high) | 3 × 10⁶cells/patient | 5 | I.V. | 1 dose |
| | | | IgG4 | 1.1 mg/kg | 5 | S.C. | Q10D x 3 |
| G4 | F | 6 | CAR-T (low) | 3 × 10⁵cells/head | 5 | I.V. | Episode 1 |
| | | | GI-101A | 3.0 mg/kg | 5 | S.C. | Q10D x 3 |
| G5 | F | 6 | CAR-T (high) | 3 × 10⁶cells/head | 5 | I.V. | 1 dose |
| | | | GI-101A | 3.0 mg/kg | 5 | S.C. | Q10D x 3 |

T cells expressing the CAR gene (CD19scFv-CD28-CD3ζ) (ProMab Biotechnologies, Cat. No. PM-CAR1001) were administered to a lymphoma xenograft mouse, followed by administration of IgG4-Fc and GI-101A at 10-day intervals from the day after administration through day 33. Body weight was measured regularly during the study period.

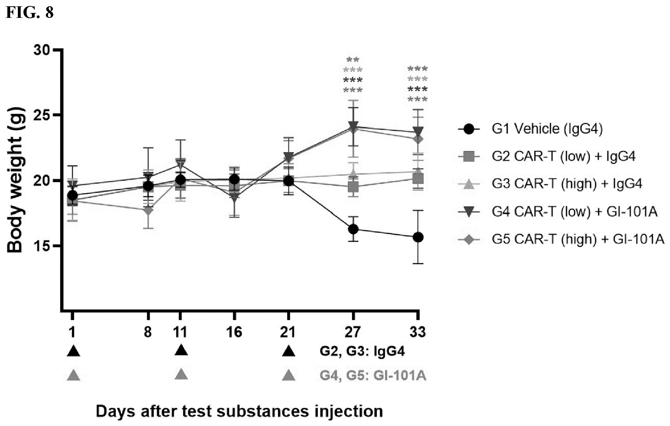

Body weight measurements taken during the study period (FIG. 8) showed that the body weight of the control group G1 Vehicle (IgG4 1.1 mg/kg) gradually increased but began to decrease from day 16. As a result, a statistically significant difference in body weight was observed between the groups administered with CAR-T and the test substances and G1 starting from day 27. In terms of body weight changes, the groups administered with CAR-T cells showed a greater tendency toward weight gain compared to the untreated G1 group, and this was observed regardless of the number of CAR-T cells administered.

The total bioluminescence flux, reflecting tumor growth from days 0 to 33, were significantly lower from day 7 to day 32 in all groups administered CAR-T and the test substance compared to the control group G1.

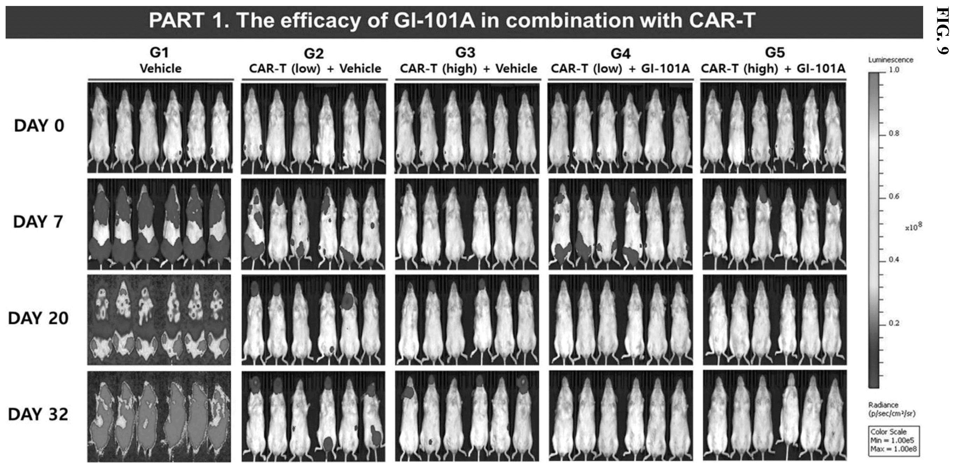

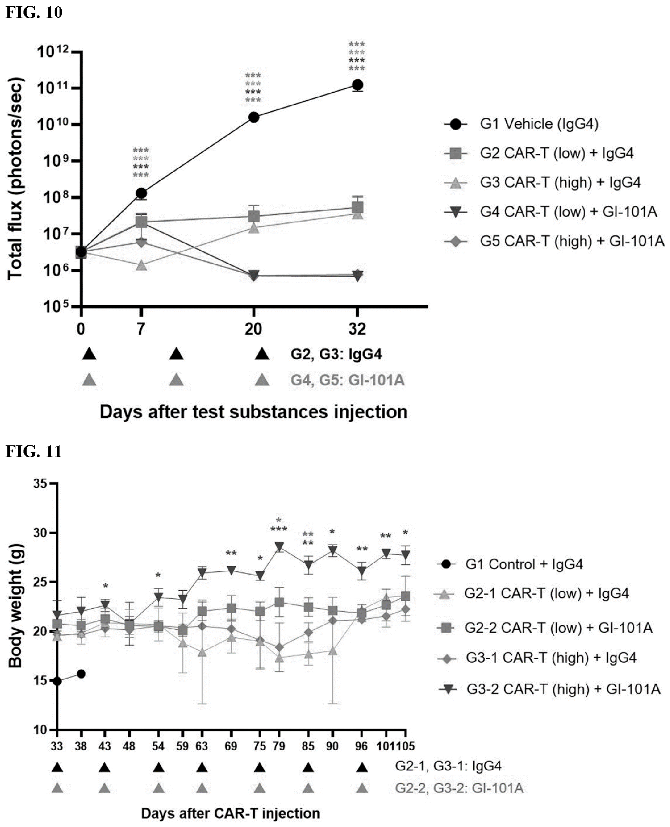

Bioluminescence imaging showed that groups administered high-dose (3 × 10⁶ cells) CAR-T cells (G3 and G5) exhibited better antitumor effects than groups administered low-dose (3 × 10⁵ cells) CAR-T cells (G2 and G4) (FIGS. 9 and 10). Regarding clinical symptoms, in the Vehicle group (G1), symptoms such as a crawling posture, hypothermia and weakness appeared starting on day 21, and by day 33, all subjects exhibited these symptoms. These symptoms were not observed in the CAR-T treatment groups (G2, G3, G4, G5), suggesting a protective or therapeutic effect of CAR-T therapy. Furthermore, bioluminescence imaging revealed that GI-101A exhibited superior antitumor effects compared to the vehicle (IgG4), and in some animals in the GI-101A-treated groups (G4 and G5), tumors were completely eliminated. Therefore, starting on day 33, each group was divided into two subgroups of three animals each, and the study continued.

### Example 4.3. Administration of Test Substance and Tumor Growth Evaluation (Part 2)

In the Part 1 study, the G2 and G3 groups, which had received IgG4 in combination with CAR-T, were divided into two subgroups and administered GI-101A or IgG4 under the conditions shown in Table 4, with monitoring continuing until day 105. This phase was defined as the Part 2 study.

**[Table 4]**

| Group | Sex | Number of Subjects | Test Substance | Administration | | | |
|---|---|---|---|---|---|---|---|
| Group | Sex | Number of subjects | Test substance | Dose | Volume (mL/kg) | Route | Cycle |
| G1 | F | 6 | IgG4 | 1.1 | 5 | S.C | - |
| G2-1 | F | 3 | IgG4 | 1.1 | 5 | S.C | Q10D x 7 |
| G2-2 | F | 3 | GI-101A | 3.0 | 5 | S.C | Q10D x 7 |
| G3-1 | F | 3 | IgG4 | 1.1 | 5 | S.C | Q10D x 7 |
| G3-2 | F | 3 | GI-101A | 3.0 | 5 | S.C | Q10D x 7 |

Weight measurements taken during the study revealed significant differences in weight changes between the treatment groups (FIG. 11). In particular, the groups administered GI-101A (G2-2 and G3-2) showed sustained weight gain compared to the groups administered IgG4 (G2-1 and G3-1), and this effect was more pronounced in the group (G3-2) that received a large number of CAR-T cells in Part 1.

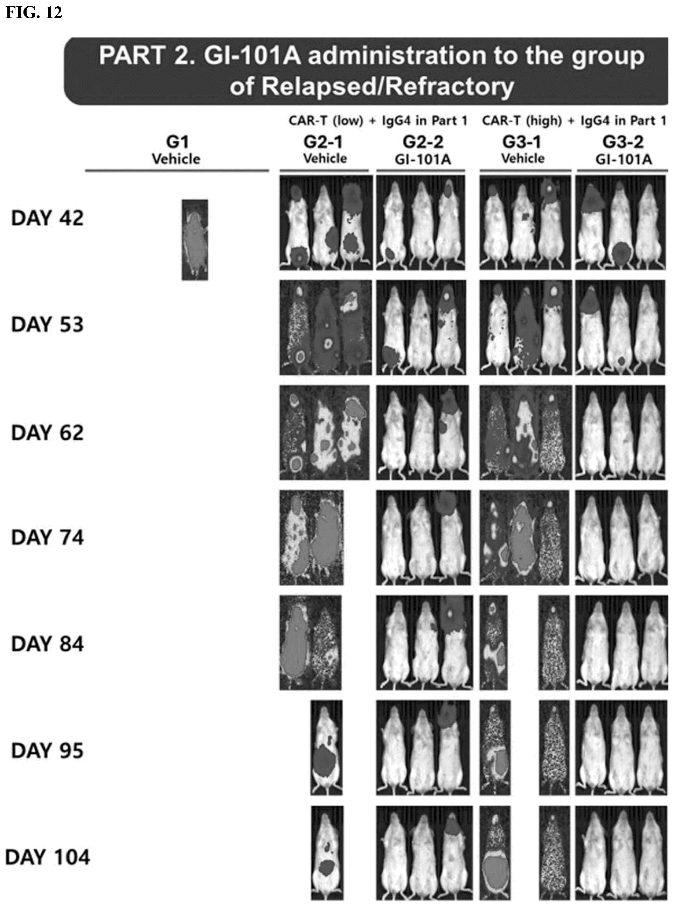

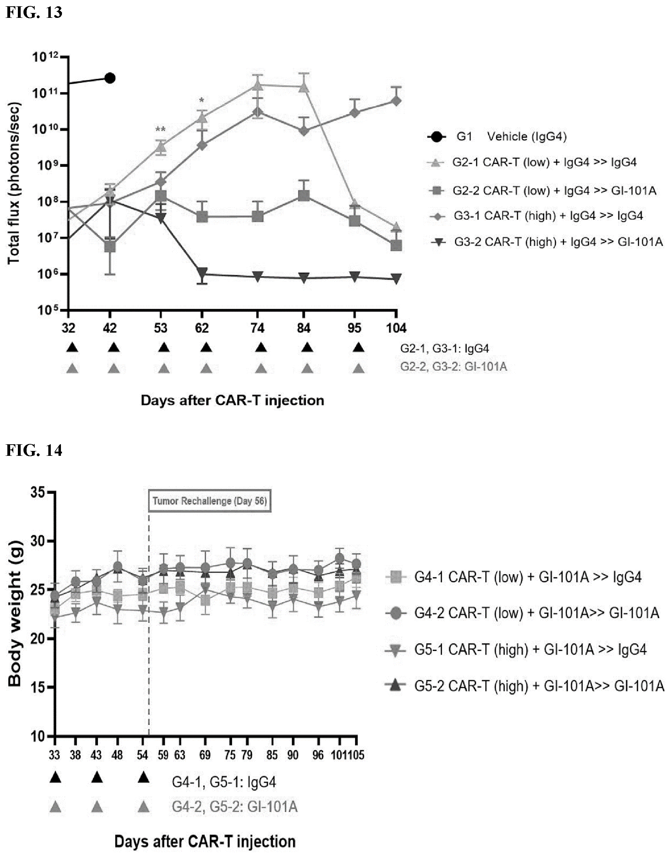

In terms of tumor growth assessed via bioluminescence imaging, the GI-101A-treated groups (G2-2 and G3-2) showed an increase in tumor suppression over time (FIGS. 12 and 13), whereas the IgG4-treated groups (G2-1 and G3-1) exhibited hypothermia and signs of weakness before the animals died. Similar to the body weight gain results, these findings demonstrated superior antitumor effects in the group that received a high dose (3 ×10⁶ cells) of CAR-T cells in Part 1 (G3-2). The results obtained in the Part 2 study emphasize that combination therapy plays a crucial role in achieving better cancer treatment outcomes.

### Example 4.4. Administration of Test Substance and Tumor Growth Evaluation (Part 3)

Groups G4 and G5, which received GI-101A in combination with CAR-T in the Part 1 study, were each divided into two subgroups. These subgroups received GI-101A or IgG4 according to the conditions in Table 5 until Day 55, underwent re-infusion of Daudi-Luc cells on Day 56, and were monitored until Day 105.

**[Table 5]**

| Group | Gender | Number of Individuals | Days 33-55 | | | | | Day 56 |
|---|---|---|---|---|---|---|---|---|
| Group | Gender | Number of subjects | Test substance | Administration | | | | Tumor Reimplantation (Daudi-Luc) |
| Group | Sex | Number of subjects | Test substance | Dose | Volume (mL/kg) | Route | Cycle | Tumor Reimplantation (Daudi-Luc) |
| G4-1 | F | 3 | IgG4 | 1.1 | 5 | S.C. | Q10D x 3 | 5 × 10⁵cells/head |
| G4-2 | F | 3 | GI-101A | 3.0 | 5 | S.C. | Q10D x 3 | 5 x 10 ⁵cells/head |
| G5-1 | F | 3 | IgG4 | 1.1 | 5 | S.C. | Q10D x 3 | 5 × 10⁵cells/head |
| G5-2 | F | 3 | GI-101A | 3.0 | 5 | S.C. | Q10D x 3 | 5 x 10⁵cells/head |

Body weight measurements taken during the study period showed no significant differences between G4-2 and G4-1, or between G5-2 and G5-1 (FIG. 14).

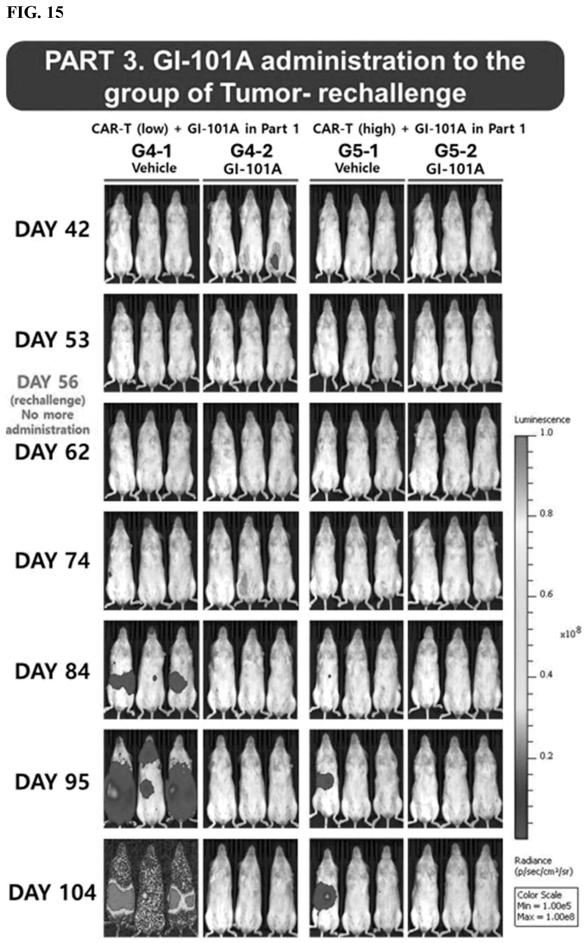

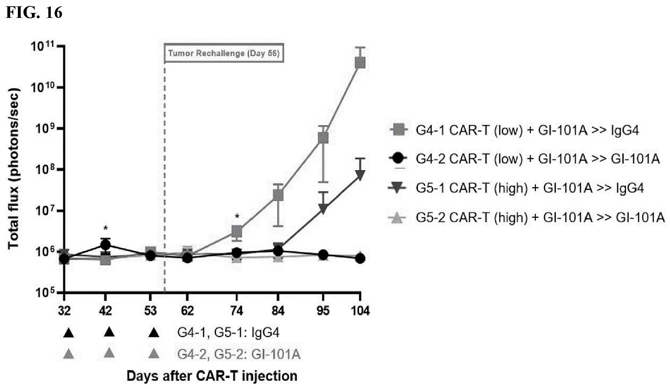

According to bioluminescence imaging results, some animals that still had tumors at the start of Part 3 study showed complete tumor disappearance by Day 53, with no detectable tumors remaining in their bodies (FIGS. 15 and 16). Therefore, a tumor rechallenge was performed on Day 56 for all groups in Part 3 study, and no further dosing was administered thereafter. No changes in body weight due to the substance administered early in Part 3 were observed, nor were there any notable clinical symptoms. However, even after tumor rechallenge, no tumors were detected in the G4-2 and G5-2 groups until the end of the study. In contrast, tumors recurred over time in the G4-1 and G5-1 groups. These observations indicate that the effect of GI-101A administered to G4-2 and G5-2 in the early stages of Part 3 persisted. Therefore, this tumor recurrence was more pronounced in the group that received CAR-T cells derived from cells treated with a low dose (3 × 10⁵ cells) in Part 1 (G4-1). The results of Part 3 confirmed the sustained antitumor effect of GI-101A.

Across all three studies, the combination of GI-101A and CAR-T cells demonstrated improved health status of animals and suppression of tumor growth. Furthermore, the administration of CAR-T cells at a high dose (3 × 10⁶ cells) in combination with GI-101A during the initial treatment phase did not result in any clinical symptoms, indicating the safety of this combination regimen. Furthermore, GI-101A demonstrated potential as a maintenance therapy following CAR-T treatment.

### Example 5. The Immune-Stimulating Effects of the Combination of GI-101A and CD19-Targeted CAR-T Cells

### Example 5.1. Sample Preparation

On days 1, 11, 21, and 34 following the tumor formation analysis in Example 3.2, and on days 34, 44, 54, 63, 75, 85, and 90 following the tumor formation analysis in Examples 3.3 and 3.4, blood samples were collected from the mice in the test group on days 34, 44, 54, 63, 75, 85, 95, and 105 following the tumor formation analysis. Subsequently, FACS analysis was performed on CD4+ T cells and CD8+ T cells using the blood samples.

Specifically, 5 mL of RBC Lysis Buffer (eBioscience, Cat. No. 00-4300-54) was added to 200 µL of blood sample, and the mixture was allowed to react at room temperature for 6 minutes. Serum-free RPMI medium (Gibco, Cat. No. A1049101) was added to the sample, and it was centrifuged at 500×g for 5 minutes. The supernatant was removed, and 5 mL of RBC Lysis Buffer was added again and allowed to react for 6 minutes. The sample was then resuspended in serum-free RPMI medium and centrifuged at 500×g for 5 minutes. Subsequently, the cells were resuspended in 100-200 µL of FACS buffer [PBS buffer containing 2% HI-FBS (heat-inactivated FBS)]. Cell counts were measured using the ADAM cell counter system (NanoEntec).

After addition of 50 µL human Fc blocker to the FACS tube and incubating at room temperature (RT) for 15 minutes, the antibody cocktail for cell surface staining (Alexa Flour 700 anti-mouse CD45 antibody (Biolegend, Cat. No. 103128), BV510 anti-human CD45 antibody (Biolegend, Cat. No. 368526), PerCP anti-human CD19 antibody (BD, Cat. No. 552851), and PerCP-Cy5.5 anti-human CD3 antibody (BD, Cat. No. 560835), BV785 anti-human CD8a antibody (Biolegend, Cat. No. 301046), PE-Cy7 anti-human CD4 antibody (Biolegend, Cat. No. 317414), Fixable viability dye eFluor780 (Invitrogen, Cat. No. 65-0865-14), FITC anti-FMC63 antibody (Acrobiosystems, Cat. No. FM3-FY45P1) was added followed by incubating at 4°C for 30 minutes. Next, the cells were centrifuged for 5 minutes, the supernatant was removed, and the cells were washed twice with 200 µL of FACS buffer.

Subsequently, the cell pellet was resuspended in FACS buffer and analyzed using a Cytek Aurora flow cytometer, and the data were analyzed by FlowJo software.

### Example 5.2. Immune-enhancing effect on the Part 1 test group

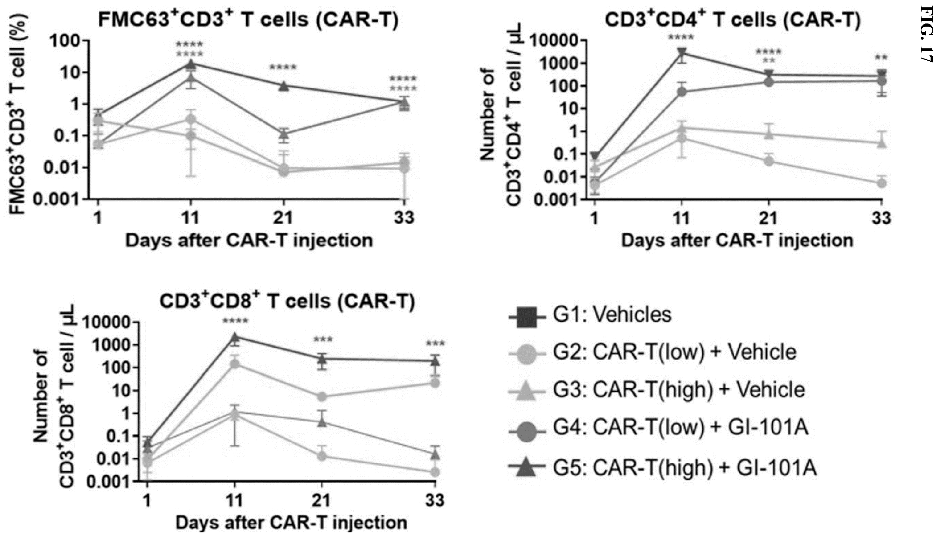

Analysis on Part 1 data revealed that in the groups treated with CAR-T cells alone (G2 and G3), the proportion of FMC63+ CD3+ T cells decreased between days 11 and 34 post-administration (FIG. 17). In the groups that received CAR-T cells in combination with GI-101A (G4 and G5), the proportion of FMC63+ CD3+ T cells among total cells was higher than in the CAR-T monotherapy groups (G2 and G3). In Group G4, the proportion of FMC63+ CD3+ T cells decreased on Day 21; however, Groups G4 and G5 maintained a higher proportion of FMC63+ CD3+ T cells than Groups G2 and G3 until Day 34. The numbers of CD4+ T cells and CD8+ T cells were found to be higher in the group receiving the combination of CAR-T and GI-101A than in the group receiving CAR-T alone and the control group (G2 vs. G4, G3 vs. G5).

### Example 5.3. CAR-T cell reactivation following GI-101A administration in mice with cancer recurrence after CAR-T cell administration (Analysis of the immune-activating effect on the Part 2 group)

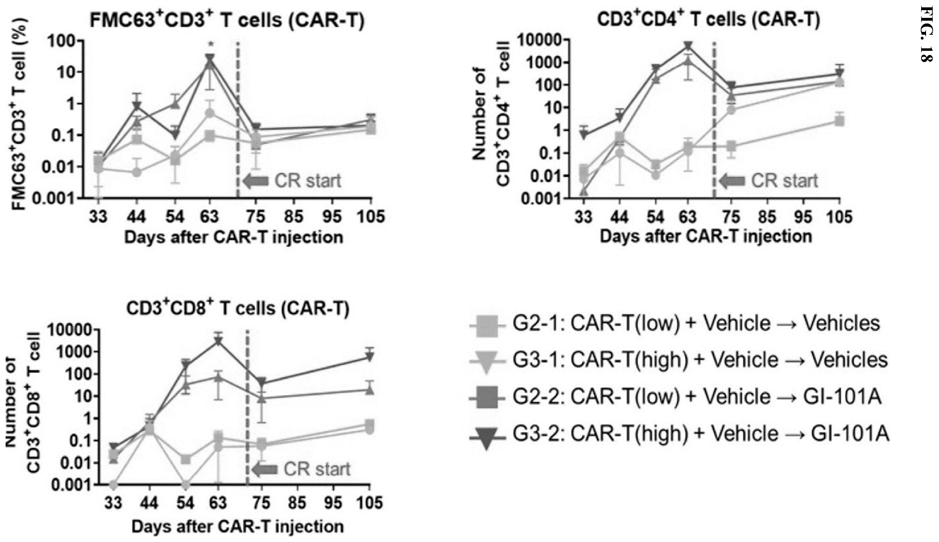

Analysis on the Part 2 study showed that the proportion of CAR-T cells increased in the group administered GI-101A, and the proportion of CAR-T cells decreased after Day 63, when complete remission was observed (FIG. 18). In the G2-2 and G3-2 groups treated with GI-101A, the number of CD4+ T cells and CD8+ T cells increased compared to the control groups (G2-1 and G3-1) until the end of the study. In particular, starting from day 54, there was a difference in the number of CD4+ T cells and CD8+ T cells between the GI-101A-treated group and the IgG4-treated group. Furthermore, in the G2-2 and G3-2 groups that continued to receive GI-101A, the number of CD4+ and CD8+ T cells was maintained until the end of the experiment on day 105. In one mouse from the G2-1 group, tumor regrowth and an increase in CD4+ T cell counts were observed.

### Example 5.4. Sustained CAR-T cell efficacy following cancer cell re-implantation in mice with cancer cell elimination after CAR-T cell and GI-101A administration (Analysis of immune-activating effects on Part 3 groups)

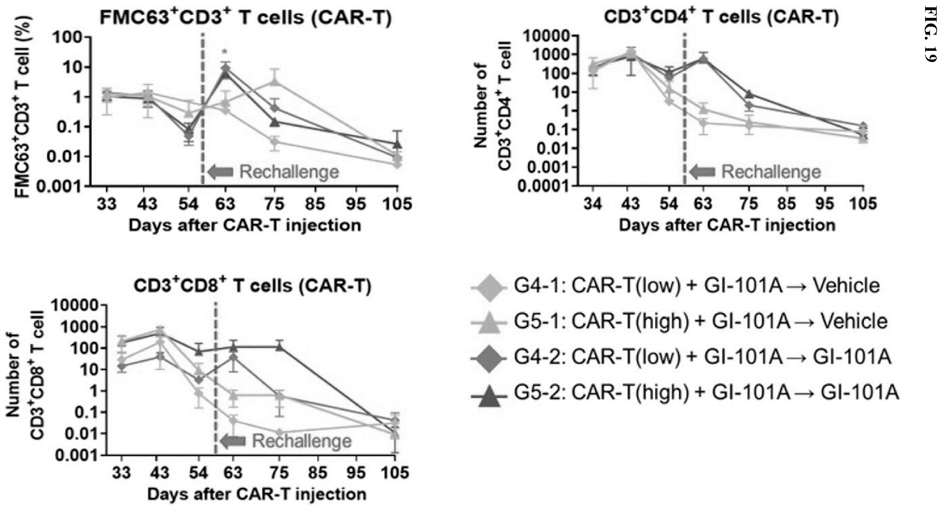

Analysis on the Part 3 study revealed that in the G4-2 and G5-2 groups, the proportion of FMC63+ CD3+ T cells increased sharply on day 63 after Luc-daudi re-injection (FIG. 19). Consistent with this result, the levels of CD4+ T cells and CD8+ T cells also increased. The increased CD4+ T cells and CD8+ T cells gradually decreased until the end of the test, which is associated with the maintenance of complete remission (CR) in the tumor.

The combination of CAR-T cell and GI-101A, as well as the additional administration of GI-101A upon relapse, significantly enhances the expansion and persistence of CAR-T cells. These results suggest that GI-101A can enhance the antitumor efficacy of CAR-T cell therapy and may serve as a promising strategy for improving clinical outcomes in patients with lymphoid malignancies. Furthermore, GI-101A demonstrates potential as a maintenance therapy following CAR-T treatment.

### Example 6. The effects of GI-102 on reactivating CD19-targeted CAR-T cells and enhancing their tumor-killing capacity in a lymphoma xenograft mouse model

### Example 6.1. Generation of a lymphoma NSG mouse model and isolation of experimental groups

To establish a lymphoma xenograft mouse model, B-Luc Daudi cancer cells expressing luciferase were injected into the tail veins of 4-8-week-old NSG mice (NOD scid gamma mouse, Jackson Labs) at a dose of 5 x 10⁵ cells/200 µL per mouse. Following the administration of B-Luc Daudi cells, *in vivo* imaging was performed 7 days prior to CAR-T cell injection. Bioluminescence intensity comparable across groups was confirmed using the IVIS Lumina III (PerkinElmer, Inc.), after which the mice were classified into groups. To measure bioluminescence, luciferin was administered intraperitoneally 10 minutes prior to *in vivo* imaging. Animals were maintained under isoflurane-inhalation anesthesia during imaging. After *in vivo* imaging, regions of interest (ROIs) were designated for each subject, and photon counts within those regions were recorded. Bioluminescence was measured on days 0, 7, 20, 30, 40, 50, 66, 77, 87, and 95 to monitor the progress of the experiment.

Mice in good health were randomly selected based on their physiological condition, body weight changes, and tumor growth rate. The selected animals were divided into groups of 7 per group, as evenly as possible, based on tumor formation and body weight.

### Example 6.2. Administration of Test Substance and Tumor Growth Evaluation (Part 1)

The test groups were configured as shown in Table 6 below, and the test substance was administered. The period from the CAR-T administration date (Day 0) to the test substance administration date (Day 1) through Day 31 was defined as Part 1.

**[Table 6]**

| Group | Sex | Number of Subjects | Test Substance | Administration | | | |
|---|---|---|---|---|---|---|---|
| | | | | Dose | Volume (mL/kg) | Route | Frequency |
| G1 | F | 7 | IgG4 | 1.1 mg/kg | 5 | S.C. | Once every 10 days (Q10D) x 3 |
| G2 | F | 7 | CAR-T (low) | 3 × 10⁵cells/patient | 5 | I.V. | 1 dose |
| | | | IgG4 | 1.1 mg/kg | 5 | S.C. | Q10D x 3 |
| G3 | F | 7 | CAR-T (high) | 3 × 10⁶cells/patient | 5 | I.V. | 1 dose |
| | | | IgG4 | 1.1 mg/kg | 5 | S.C. | Q10D x 3 |
| G4 | F | 7 | CAR-T (low) | 3 × 10⁵cells/head | 5 | I.V. | 1 dose |
| | | | GI-102 | 3.0 mg/kg | 5 | S.C. | Q10D x 3 |
| G5 | F | 7 | CAR-T (high) | 3 × 10⁶cells/patient | 5 | I.V. | 1 dose |
| | | | GI-102 | 3.0 mg/kg | 5 | S.C. | Q10D x 3 |

T cells (ProMab Biotechnologies, Cat. No. PM-CAR1001) expressing the CAR gene (CD19scFv-CD28-CD3ζ) were administered to a lymphoma xenograft mouse model, followed by administration of IgG4-Fc and GI-102 at 10-day intervals from the day after administration through Day 31.

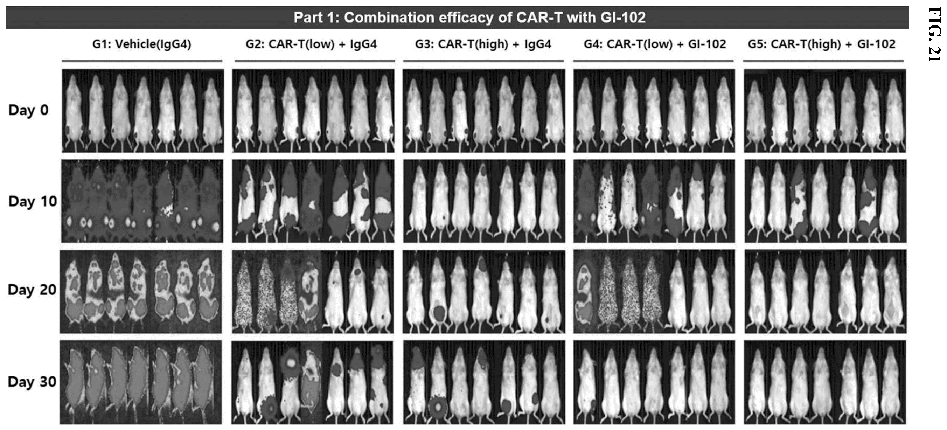

The total bioluminescence flux, which measured tumor growth from days 0 to 31, was significantly lower in all groups treated with CAR-T and the test substances from day 10 to day 30 compared to the control group G1 (FIG. 21).

Bioluminescence imaging results showed that the groups (G3 and G5) administered high-dose (3 x 10⁶ cells) CAR-T cells exhibited better antitumor effects than the groups (G2 and G4) administered low-dose (3 x 10⁵ cells) CAR-T cells. Furthermore, bioluminescence imaging revealed that GI-102 exhibited superior antitumor effects compared to IgG4 Fc; in some individuals in the group (G4) that received a small number of CAR-T cells and GI-102, tumors completely disappeared after day 20, and in all subjects in the group (G5) that received a high dose of CAR-T and GI-102, tumors completely disappeared after day 20.

### Example 6.3. Administration of Test Substances and Tumor Growth Evaluation (Part 2)

The G2 and G3 groups, which received IgG4 along with CAR-T in Part 1 of the study, and the G4 and G5 groups, which received GI-102 along with CAR-T, were divided into two subgroups. GI-102 or IgG4 was administered and monitored under the conditions shown in Table 7. This phase was defined as Part 2 study.

**[Table 7]**

| Group | Sex | Number of subjects | Test substance | Administration | | | |
|---|---|---|---|---|---|---|---|
| | | | | Dose | Volume (mL/kg) | Route | Cycle |
| G2-1 | F | 3 | IgG4 | 1.1 | 5 | S.C. | Q10D x 6 |
| G2-2 | F | 4 | GI-102 | 3.0 | 5 | S.C. | Q10D x 6 |
| G3-1 | F | 3 | IgG4 | 1.1 | 5 | S.C. | Q10D x 6 |
| G3-2 | F | 4 | GI-102 | 3.0 | 5 | S.C | Q10D x 6 |
| G4-1 | F | 3 | IgG4 | 1.1 | 5 | S.C. | Q10D x 3 |
| G4-2 | F | 4 | GI-102 | 3.0 | 5 | S.C. | Q10D x 3 |
| G5-1 | F | 3 | IgG4 | 1.1 | 5 | S.C. | Q10D x 3 |
| G5-2 | F | 4 | GI-102 | 3.0 | 5 | S.C | Q10D x 3 |

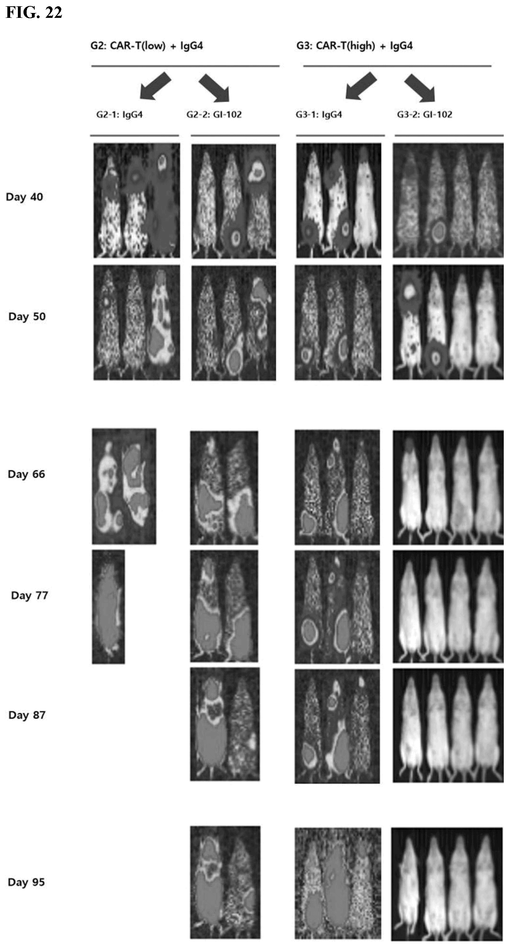

In view of tumor growth assessed by bioluminescence imaging, the tumor suppression effect increased over time in the group administered GI-102 (G3-2) (FIG. 22). The group that received a large number of CAR-T cells in Part 1 (G3-2) demonstrated a superior antitumor effect.

### Example 6.4. Administration of Test Substances and Tumor Growth Evaluation (Part 3)

In the Part 1 study, the G4 and G5 groups, which received GI-102 along with CAR-T, were each divided into two subgroups. GI-102 or IgG4 was administered according to the conditions in Table 8 until Day 58, Daudi-Luc cells were re-implanted on Day 59, and the animals were monitored until Day 87.

**[Table 8]**

| Group | Sex | Number of Subjects | Days 31-58 | | | | | Day 59 |
|---|---|---|---|---|---|---|---|---|
| | | | Test substance | Administration | | | | Tumor Reimplantation (Daudi-Luc) |
| | | | | Dose (mg/kg) | Volume (mL/kg) | Route | Cycle | |
| G4-1 | F | 3 | IgG4 | 1.1 | 5 | S.C. | Q10D x 3 | 5 x 10⁵cells/head |
| G4-2 | F | 4 | GI-102 | 3.0 | 5 | S.C. | Q10D x 3 | 5 x 10⁵cells/head |
| G5-1 | F | 3 | IgG4 | 1.1 | 5 | S.C. | Q10D x 3 | 5 x 10⁵cells/head |
| G5-2 | F | 4 | GI-102 | 3.0 | 5 | S.C. | Q10D x 3 | 5 x 10⁵cells/head |

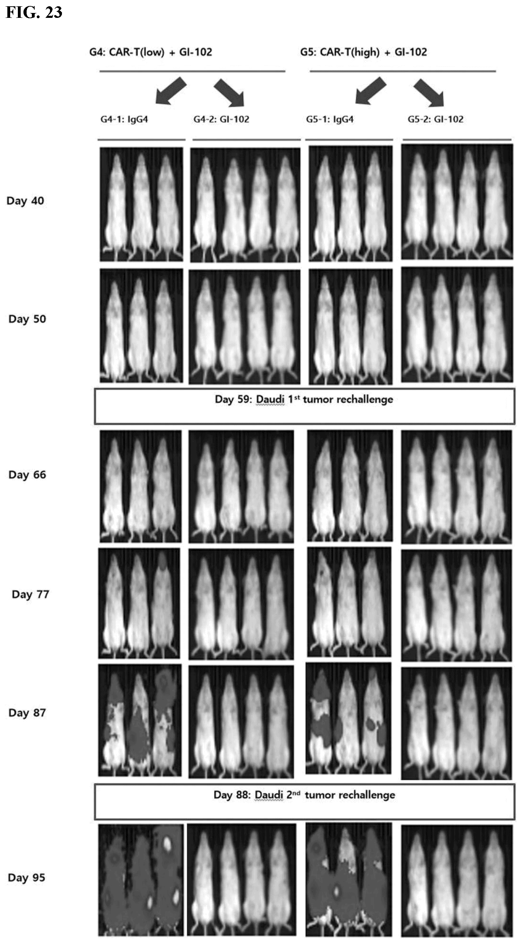

Bioluminescence imaging results showed that no detectable tumors remained in any of the subjects from the start of Part 3 study through Day 50 (FIG. 23). Tumor re-implantation was performed on Day 59 for all groups in Part 3 study. Even after tumor re-implantation, no tumors were detected in any group through Day 66. In contrast, tumors recurred over time in the G4-1 and G5-1 groups. These observations indicate that the effect of GI-102 administered to G4-2 and G5-2 in the early stages of Part 3 persisted. The results of Part 3 confirmed the sustained antitumor effect of GI-102.

### Example 6.5. Administration of Test Substance and Tumor Growth Evaluation (Part 4)

In the Part 3 study, although tumors were reimplanted on Day 59 in the G4-2 and G5-2 groups, no tumors were detected until Day 87. Consequently, a second tumor reimplantation was performed on Day 88, and monitoring was conducted until Day 95; however, no tumors were detected by the end of the study (FIG. 23). The results of Part 4 confirmed the sustained antitumor effect of GI-102.

**[Table 9]**

| Group | Sex | Number of subjects | Day 88 |
|---|---|---|---|
| | | | Tumor Reimplantation (Daudi-Luc) |
| G4-1 | F | 3 | 5 x 10⁵cells/head |
| G4-2 | F | 4 | 5 x 10⁵cells/head |
| G5-1 | F | 3 | 5 x 10⁵cells/head |
| G5-2 | F | 4 | 5 x 10⁵cells/head |

Throughout the tests up to Part 4, the combination of GI-102 and CAR-T cells demonstrated that it improves the health status of subjects and suppresses tumor growth. Furthermore, the combination of CAR-T cells and GI-102 administered during the initial treatment phase demonstrated excellent tumor growth inhibition efficacy. It was confirmed that antitumor efficacy was maintained even without additional administration of GI-102, thereby demonstrating that GI-102 can be used as a maintenance therapy following CAR-T treatment.

### Example 7. The immune-activating effects of the combination of GI-102 and CD19-targeted CAR-T cells

### Example 7.1. Sample Preparation

Blood samples were collected from mice in the test group on day 11 after the tumor formation assay in Example 6.2, and on day 52 after the tumor formation assay in Examples 6.3 and 6.4. Subsequently, FACS analysis was performed on CD4+ T cells and CD8+ T cells using these blood samples.

The specific experimental methods were the same as those described in Example 5.1.

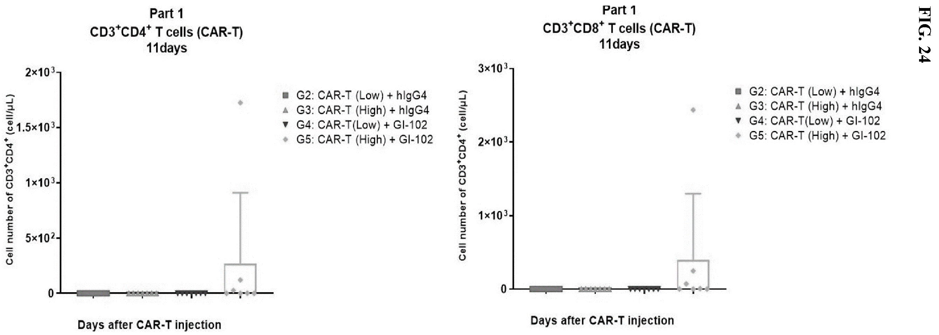

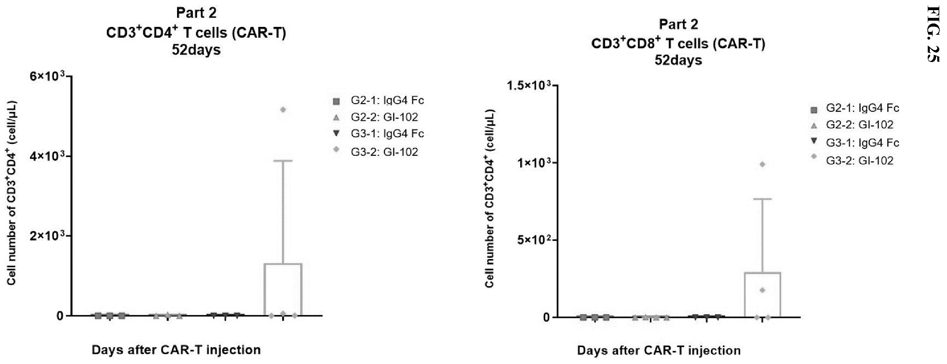

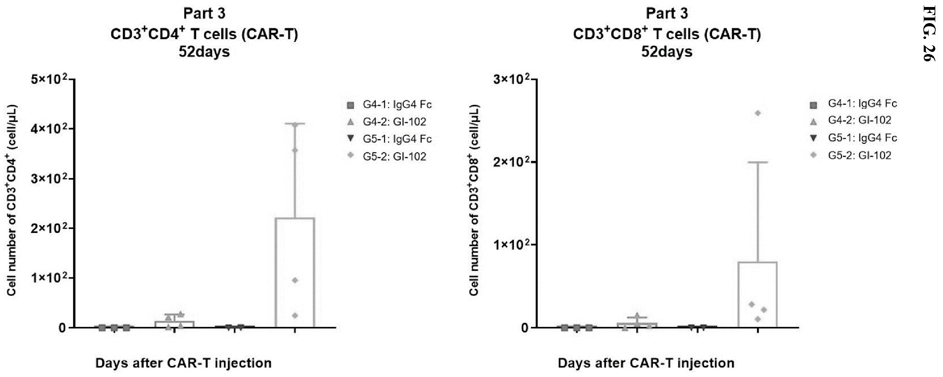

The FACS analysis results showed that only in the group that received a large number of CAR-T cells and GI-102 in Part 1 study (G5), the proportions of CD4+ T cells and CD8+ T cells increased on day 11 post-administration (FIG. 24). Furthermore, in Part 2 study, the proportion of CD4+ T cells and CD8+ T cells increased on day 52 only in the group that received a large number of CAR-T cells in Part 1 and an additional dose of GI-102 (G3-2) (FIG. 25). In the Part 3 trial, the ratio of CD4+ T cells and CD8+ T cells increased at Day 52 only in the group that received a high dose of CAR-T cells and GI-102 in the Part 1 test and subsequently received an additional dose of GI-102 (G5-2) (FIG. 26).

The combination of CAR-T cell and GI-102, as well as the additional administration of GI-102 upon relapse, significantly enhances the expansion and persistence of CAR-T cells. These results suggest that GI-102 may serve as a promising strategy to enhance the antitumor efficacy of CAR-T cell therapy and improve clinical outcomes for patients with lymphoid malignancies. Furthermore, GI-102 demonstrates potential as a maintenance therapy following CAR-T therapy.

### Example 8. The effects of GI-102 on reactivating BCMA-targeted CAR-T cells and enhancing their tumor-killing ability in a multiple myeloma mouse model

### Example 8.1. Establishment of a multiple myeloma mouse model and isolation of experimental groups

A multiple myeloma xenograft mouse model was established by intravenously injecting 5 x 10⁶ U266 (human multiple myeloma cells) expressing luciferase into the tail vein of NSG mice aged 4-8 weeks. Following intravenous injection, D-luciferin dissolved in PBS was administered intraperitoneally, and 15-20 minutes later, the degree of fluorescence was measured using an in vivo imaging system (IVIS) to confirm engraftment. The animals were maintained under isoflurane-inhalation anesthesia during imaging. Following *in vivo* imaging, regions of interest (ROIs) were designated for each subject, and photon counts within these regions were recorded. When tumor formation was confirmed via biofluorescence analysis 14 days later, T cells engineered with the CAR gene (BCMAscFv-41BB-CD3z) (ProMab Biotechnologies, Cat. No. PM-CAR1037, 1×10⁶/ head) were administered intravenously (day 0). The following day, GI-102 or IgG4 was administered (day 1), and tumor formation was monitored by measuring flux values via bioluminescence analysis. Bioluminescence was measured on days 0, 7, 16, and 23 to monitor the progress of the experiment.

In the established multiple myeloma model, body weight and adverse reactions were monitored twice weekly for each individual.

### Example 8.2. Administration of Test Substance and Tumor Growth Evaluation

Test groups were established and test substances were administered as shown in Table 10 below.

**[Table 10]**

| Group | Test Substance | Administration | | | |
|---|---|---|---|---|---|
| | | Dose | Volume (mL/kg) | Route | Cycle |
| G1 | IgG4 | 1.1 mg/kg | 5 | S.C. | Once on day 8 and once on day 17 |
| G2 | CAR-T | 1 × 10⁶cells/patient | 5 | I.V. | Once on Day 0 |
| G2 | IgG4 | 1.1 mg/kg | 5 | S.C. | Once on day 8 and once on day 17 |
| G3 | CAR-T | 1 × 10⁶cells/patient | 5 | I.V. | Once on Day 0 |
| G3 | GI-102 | 3.0 mg/kg | 5 | S.C. | Once on days 8 and once on day 17 |

Following administration of CAR-T cells to a multiple myeloma mouse model, IgG4-Fc and GI-102 were administered on days 7 and 16, respectively.

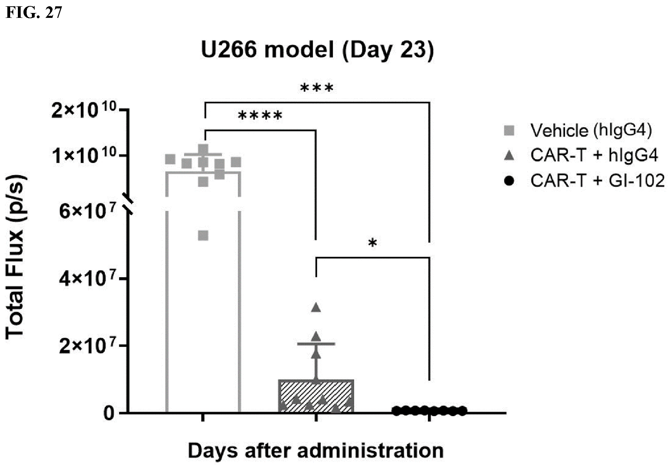

The total bioluminescence flux, which measures tumor growth, was significantly reduced in the group receiving CAR-T and GI-102 in combination (G3) compared to the group receiving CAR-T alone (CAR-T + hIgG4, G2) (FIG. 27).

No weight loss or specific toxic symptoms were observed following administration of the agents.

### Example 9. Analysis of the immunostimulatory effect of the combination of GI-102 and BCMA-targeted CAR-T

As described in Example 8.2 above, blood samples were collected from mice in the test group on day 24 after T-cell administration. Subsequently, FACS analysis was performed on CD4+ T cells and CD8+ T cells using the blood samples.

The specific experimental methods were the same as those described in Example 5.1.

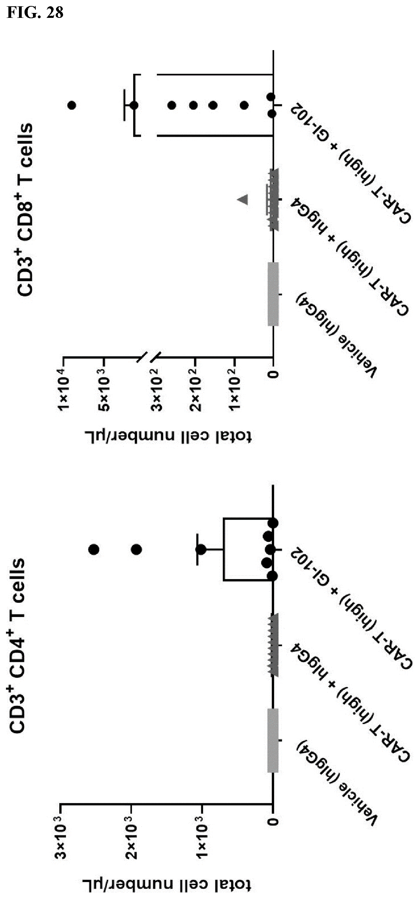

FACS analysis revealed that the proportions of CD4+ T cells and CD8+ T cells increased only in the group that received CAR-T cells and GI-102 (FIG. 28).

**[Table 11]**

| **ScFv** | Domain | Amino Acid Sequence **(Sequence Number)** | | | |
|---|---|---|---|---|---|
| | | Variable Region | CDR1 | CDR2 | CDR3 |
| Anti-CD19 ScFv (Promab) | **VH** | EVQLVESGGGLVQPGGSLRL SCAASGVSLPDYGVSWVRQ APGKGLEWVSVIWGSETTY YNSALKSRFTISRDNSKNTLY LQMNSLRAEDTAVYYCAKH YYYGGSYAMDYWGQGTLV TVSS (32) | GVSLPDY GVS (29) | VIWGSETT YYNSALK (30) | KHYYYGG SYAMDY (31) |
| | **VL** | DIQMTQSPSSLSASVGDRVTI TCRASQDISKYLNWYQQKP GKAPKLLIYHTSRLHSGVPSR FSGSGSGTDFTLTISSLQPEDF ATYYCQQGNTLPYTFGGGT KVEIK (36) | RASQDISK YLN (33) | HTSRLH **(34)** | QQGNTLP Y (35) |
| | **Linker** | GSTSGSGKPGSGEGSTKG **(37)** | | | |
| Anti-CD19 ScFv (Kymriah) | **VH** | EVKLQESGPGLVAPSQSLSV TCTVSGVSLPDYGVSWIRQP PRKGLEWLGVIWGSETTYY NSALKSRLTIIKDNSKSQVFL KMNSLQTDDTAIYYCAKHY YYGGSYAMDYWGQGTSVT VSS (41) | GVSLPDY GVS (38) | VIWGSETT YYNSALK (39) | KHYYYGG SYAMDY (40) |
| | **VL** | DIQMTQTTSSLSASLGDRVTI SCRASQDISKYLNWYQQKPD GTVKLLIYHTSRLHSGVPSRF SGSGSGTDYSLTISNLEQEDI ATYFCQQGNTLPYTFGGGTK LEIT (45) | RASQDISK YLN (42) | HTSRLH **(43)** | QQGNTLP Y (44) |
| | **Linker** | GGGGSGGGGSGGGGS **(46)** | | | |
| Anti-CD19 ScFv (Yescarta) | **VH** | EVQLVESGGGLVQPGRSLRL SCTASGVSLPDYGVSWIRQP PGKGLEWIGVIWGSETTYYN SALKSRFTISRDNSKNTLYLQ MNSLRAEDTAVYYCAKHYY YGGSYAMDYWGQGTLVTV SS (50) | GVSLPDY **(47)** | VIWGSETT YYNSALK S (48) | HYYYGGS YAMDY (49) |
| | **VL** | DIQMTQSPSSLSASVGDRVTI TCRASQDISKYLNWYQQKP DQAPKLLIKHTSRLHSGVPSR FSGSGSGTDYTLTISSLQPED FATYYCQQGNTLPYTFGQGT KLEIK (54) | RASQDISK YLN (51) | HTSRLHS **(52)** | QQGNTLP YT (53) |
| | **Linker** | GSTSGSGKPGSGEGSTKG **(55)** | | | |
| Anti-BCMA ScFv (Promab) | **VH** | DIVMTQSPATLSVTPGDRVS LSCRASQSISDYLHWYQQKS HESPRLLIKYASQSISGIPSRF SGSGSGSDFTLSINSVEPEDV GVYYCQNGHSFPPTFGGGTK LEIK (56) | - | - | - |
| | **VL** | VQLQQSGPELVKPGASVKM SCKASGYTFTSYVMHWVKQ KPGQGLEWIGYIIPYNDATK YNEKFKGKATLTSDKSSSTA YMELSSLTSEDSAVYYCARY NYDGYFDVWGAGTTVTVSS (57) | - | - | - |
| | **Linker** | GGGGSGGGGSGGGGS **(58)** | | | |
| Anti-BCMA ScFv (Abecma) | **VH** | QIQLVQSGPELKKPGETVKIS CKASGYTFTDYSINWVKRAP GKGLKWMGWINTETREPAY AYDFRGRFAFSLETSASTAY LQINNLKYEDTATYFCALDY SYAMDYWGQGTSVTVSS (62) | DYSIN **(59)** | WINTETRE PAYAYDF RG (60) | DYSYAMD Y (61) |
| | **VL** | MALPVTALLLPLALLLHAAR PDIVLTQSPPSLAMSLGKRAT ISCRASESVTILGSHLIHWYQ QKPGQPPTLLIQLASNVQTG VPARFSGSGSRTDFTLTIDPV EEDDVAVYYCLQSRTIPRTF GGGTKLEIK (66) | RASESVTI LGSHLIH (63) | LASNVQT **(64)** | LQSRTIPR T (65) |
| | **Linker** | GSTSGSGKPGSGEGSTKG **(67)** | | | |
| Anti-BCMA ScFv (Promab_ CAR1037) | **VH** | EIVLTQSPATLSLSPGERATL SCRASQSISDYLHWYQQKPG QAPRLLIYYASQSITGIPARFS GSGSGTDFTLTISSLEPEDFA VYYCQNGHSFPPTFGGGTKV EIKLE (68) | - | - | - |
| | **VL** | MALPVTALLLPLALLLHAAR PASQVQLVQSGAEVKKPGSS VKVSCKASGYTFTSYVMHW VRQAPGQGLEWMGYIIPYN DATKYAQKFQGRVTITADKS TSTAYMELSSLRSEDTAVYY CARYNYDGYFDVWGQGTL VTVSS (69) | - | - | - |
| | **Linker** | GGGGSGGGGSGGGGS **(70)** | | | |

Having described specific embodiment of the present invention in detail above, it is to be understood that variants and modifications thereof falling within the spirit of the invention may become apparent to those skilled in this art, and the scope of this invention is to be determined by appended claims and their equivalents.

## Claims

1. A composition for enhancing the activity of an immune cell that expresses a chimeric antigen receptor, comprising as an active ingredient, a fusion protein comprising a CD80 protein or a fragment thereof and an IL-2 protein or a variant thereof.

2. The composition of claim 1, wherein the fragment of the CD80 protein is the extracellular domain of the CD80 protein.

3. The composition of claim 1, wherein the variant of the IL-2 protein comprises at least one substitution selected from the group consisting of substitutions at 38^{th}, 42^{nd} and 61^{st} amino acids in the amino acid sequence of SEQ ID NO: 10.

4. The composition of claim 1, wherein the chimeric antigen receptor comprises all or a portion of an intracellular signaling domain selected from the group consisting of CD3z, CD28, and 4-1BB.

5. The composition of claim 1, wherein the chimeric antigen receptor comprises a transmembrane domain comprising all or a portion of CD28 or CD8.

6. The composition of claim 1, wherein the chimeric antigen receptor comprises a hinge domain comprising all or a portion of the CD8 hinge.

7. The composition of claim 1, wherein the chimeric antigen receptor comprises an extracellular antigen-binding domain that binds to CD19 or BCMA.

8. The composition of claim 7, wherein the extracellular antigen-binding domain specifically binds to CD19 and comprises a heavy chain variable region comprising an HCDR1 having the amino acid sequence selected from the group consisting of SEQ ID NO: 29, SEQ ID NO: 38 and SEQ ID NO: 47; an HCDR2 having the amino acid sequence region selected from the group consisting of SEQ ID NO: 30, SEQ ID NO: 39 and SEQ ID NO: 48; and an HCDR3 having the amino acid sequence selected from the group consisting of SEQ ID NO: 31, SEQ ID NO: 40 and SEQ ID NO: 49.

9. The composition of claim 8, wherein the heavy chain variable region comprises the amino acid sequence selected from the group consisting of SEQ ID NO: 32, SEQ ID NO: 41, and SEQ ID NO: 50.

10. The composition of claim 8, wherein the extracellular antigen-binding domain further comprises a light chain variable region comprising an LCDR1 having the amino acid sequence selected from the group consisting of SEQ ID NO: 33, SEQ ID NO: 42 and SEQ ID NO: 51; an LCDR2 having the amino acid sequence region selected from the group consisting of SEQ ID NO: 34, SEQ ID NO: 43 and SEQ ID NO: 52; and an LCDR3 having the amino acid sequence selected from the group consisting of SEQ ID NO: 35, SEQ ID NO: 44 and SEQ ID NO: 53.

11. The composition of claim 10, wherein the light chain variable region comprises the amino acid sequence selected from the group consisting of SEQ ID NO: 36, SEQ ID NO: 45, and SEQ ID NO: 54.

12. The composition of claim 7, wherein the extracellular antigen-binding domain specifically binds to BCMA and comprises a heavy chain variable region comprising an HCDR1 having the amino acid sequence of SEQ ID NO: 59; an HCDR2 having the amino acid sequence of SEQ ID NO: 60; and an HCDR3 having the amino acid sequence of SEQ ID NO: 61.

13. The composition of claim 7, wherein the extracellular antigen-binding domain specifically binds to BCMA and comprises a heavy chain variable region comprising the amino acid sequence selected from the group consisting of SEQ ID NO: 56, SEQ ID NO: 62 and SEQ ID NO: 68.

14. The composition of claim 7, wherein the extracellular antigen-binding domain further comprises a light chain variable region comprising an LCDR1 having the amino acid sequence of SEQ ID NO: 63; an LCDR2 having the amino acid sequence of SEQ ID NO: 64; and an LCDR3 having the amino acid sequence of SEQ ID NO: 65.

15. The composition of claim 7, wherein the extracellular antigen-binding domain specifically binds to BCMA and comprises a light chain variable region comprising the amino acid sequence selected from the group consisting of SEQ ID NO: 57, SEQ ID NO: 66 and SEQ ID NO: 69.

16. The composition of claim 1, wherein the immune cell is selected from the group consisting of a T cell, a natural killer cell and a natural killer T (NKT) cell.

17. An anticancer composition for co-administration with an immune cell that expresses a chimeric antigen receptor, comprising as an active ingredient a fusion protein comprising the CD80 protein or a fragment thereof and the IL-2 protein or a variant thereof.

18. The anticancer composition of claim 17, wherein the anticancer composition is for preventing or treating a B-cell malignancy.

19. A pharmaceutical composition for preventing or treating cancer, comprising as active ingredients a fusion protein comprising the CD80 protein or a fragment thereof and the IL-2 protein or a variant thereof; and an immune cell that expresses a chimeric antigen receptor.

20. The composition of claim 19, wherein the cancer is a B-cell malignancy.
